# EUROPEAN PATENT SPECIFICATION

(11) **EP 3 587 418 B1**
(45) Date of publication and mention of the grant of the patent: **01.09.2021**
(21) Application number: 19183741.8
(22) Date of filing: 01.07.2019
(51) Int. Cl.: C07D 471/04, A61K 31/437, A61P 35/00

(54) **INHIBITORS OF CREB BINDING PROTEIN (CBP)**
INHIBITOREN VON CREB BINDING PROTEIN (CBP)
INHIBITEURS DE LA CREB BINDING PROTEIN (CBP)

(30) Priority: 29.06.2018 US 201862692593 P; 14.09.2018 WO PCT/US2018/051235; 14.09.2018 WO PCT/US2018/051214; 15.03.2019 US 201962819490 P; 28.06.2019 US 201916457596; 28.06.2019 WO PCT/US2019/039936
(43) Date of publication of application: 01.01.2020
(73) Proprietor: Forma Therapeutics, Inc., Watertown, MA 02472 (US)
(72) Inventor: Schiller, Shawn E. R., Watertown, MA 02472 (US); Herbertz, Torsten, Watertown, MA 02472 (US); Li, Hongbin, Watertown, MA 02472 (US); Graves, Bradford, Watertown, MA 02472 (US); Mischke, Steven, Watertown, MA 02472 (US); West, Angela V., Watertown, MA 02472 (US); Ericsson, Anna, Watertown, MA 02472 (US); Downing, Jennifer R., Watertown, MA 02472 (US)
(74) Representative: Oates, Edward Christopher

(56) References cited:
- EP-A1- 2 412 710
- WO-A1-2016/086200
- WO-A1-2019/055869
- WO-A2-2011/150156

## Description

### Technical Field

This disclosure relates to novel chemical compositions for inhibiting the CREB binding protein (CBP), useful in the treatment of treating diseases or disorders associated with the inhibition of CBP/p300 family of bromodomains.

### Background

CBP/p300 are lysine acetyltransferases that catalyze the attachment of an acetyl group to a lysine side chain of histones and other protein substrates. p300 (also known as EP300 and KAT3B) is a protein with multiple domains that bind to diverse proteins including many DNAbinding transcription factors. The cyclic AMP-responsive element-binding protein (CREB) binding protein (CBP, also known as KAT3A) is a cellular paralog of p300. p300 and CBP share extensive sequence identity and functional similarity and are often referred to as CBP/p300. CBP/p300-catalyzed acetylation of histones and other proteins is pivotal to gene activation. Heightened p300 expression and activities have been observed in advanced human cancers such as prostate and in human primary breast cancer specimens. Chemical inhibition of CBP/p300 that possesses intrinsic acetyltransferase enzymatic activity is more feasible than blocking transcription factors with small molecules, as discovery of chemical inhibitors of transcription factors has proven extremely challenging. Accordingly, there is a need for novel and potent compounds for inhibiting CBP/p300, useful as therapies for treating certain related forms of cancer.

4,5,6,7-tetrahydro-1H-pyrazolo[4,3-C]pyridin-3-amine compounds, pharmaceutical compositions comprising said compounds or a pharmaceutically acceptable salt thereof, and methods of using such compounds and salts thereof in the treatment of CBP and/or EP300 mediated disorders are described in WO 2016/086200.

### Summary

A first aspect of the present disclosure relates to compounds of Formula (I): 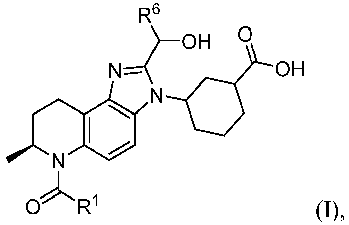 or a pharmaceutically acceptable salt, enantiomer, hydrate, solvate, stereoisomer, or tautomer thereof, wherein:
R¹ is -C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OR⁵, -N(R⁵)₂, or -NHR⁵;
R⁵ is -C₁-C₆alkyl, -C₃-C₈cycloalkyl, heterocyclyl, aryl, or heteroaryl;
R⁶ is -C₁-C₆alkyl, -C₃-C₈cycloalkyl, -C₄-C₈cycloalkenyl, heterocyclyl, aryl, spirocycloalkyl, spiroheterocyclyl, heteroaryl, halogen, oxo,-(CH₂)ₙ-OR⁸,-C(O)R^{8'}, -C(O)OR⁸, or -C(O)NR⁸R⁹, wherein each alkyl, cycloalkyl, heterocyclyl, spirocycloalkyl, spiroheterocyclyl, heteroaryl, or aryl is optionally substituted with one or more R¹⁰;
R⁸ and R⁹ are each independently, at each occurrence, -H, -C₁-C₆alkyl, -C₂-C₆alkenyl, -C₂-C₆alkynyl, -C₃-C₈cycloalkyl, -C₄-C₈cycloalkenyl, heterocyclyl, aryl, heteroaryl, wherein each alkyl, alkenyl, alkynyl, cycloalkyl, cycloalkenyl, heterocyclyl, aryl, or heteroaryl is optionally substituted with one or more R¹⁰ or R¹¹; or
R⁸ and R⁹ may combine with the atom to which they are both attached to form a spiroheterocyclyl, heterocyclyl, or heteroaryl, wherein the formed spiroheterocyclyl, heterocyclyl,or heteroaryl is optionally substituted with one or more R¹⁰ or R¹¹;
R^{8'} is each independently, at each occurrence, -C₁-C₆alkyl, -C₂-C₆alkenyl, -C₂-C₆alkynyl, -C₃-C₈cycloalkyl, -C₄-C₈cycloalkenyl, heterocyclyl, aryl, heteroaryl, wherein each alkyl, alkenyl, alkynyl, cycloalkyl, cycloalkenyl, heterocyclyl, aryl, or heteroaryl is optionally substituted with one or more R¹⁰ or R¹¹; or
R¹⁰ is each independently, at each occurrence,-C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -OC₃-C₆cycloalkyl, -Oaryl, -Oheteroaryl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl), wherein each alkyl, alkenyl, alkynyl, cycloalkyl, cycloalkenyl, heterocyclyl, heteroaryl, or aryl is optionally substituted with one or more -R¹²;
wherein any two R¹⁰ when on non-adjacent atoms, can combine to form a bridging cycloalkyl or heterocyclyl;
wherein any two R¹⁰ when on adjacent atoms, can combine to form a cycloalkyl, heterocyclyl, aryl or heteroaryl; and
R¹² is independently, at each occurrence,-C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl).

Preferably, the compounds of Formula (I) are a compound of Formula (IV): 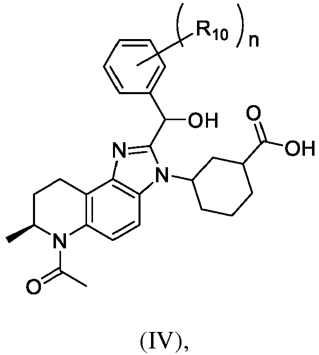 or a pharmaceutically acceptable salt thereof, wherein:
n is 0, 1, 2, 3, 4, or 5;
each R¹⁰ is independently, at each occurrence,-C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -OC₃-C₆cycloalkyl, -Oaryl, -Oheteroaryl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl), wherein each alkyl, alkenyl, alkynyl, cycloalkyl, cycloalkenyl, heterocyclyl, heteroaryl, or aryl is optionally substituted with one or more -R¹²;
wherein any two R¹⁰ when on non-adjacent atoms, can combine to form a bridging cycloalkyl or heterocyclyl;
wherein any two R¹⁰ when on adjacent atoms, can combine to form a cycloalkyl, heterocyclyl, aryl or heteroaryl; and
R¹² is independently, at each occurrence,-C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl).

### Brief Description of the Drawings

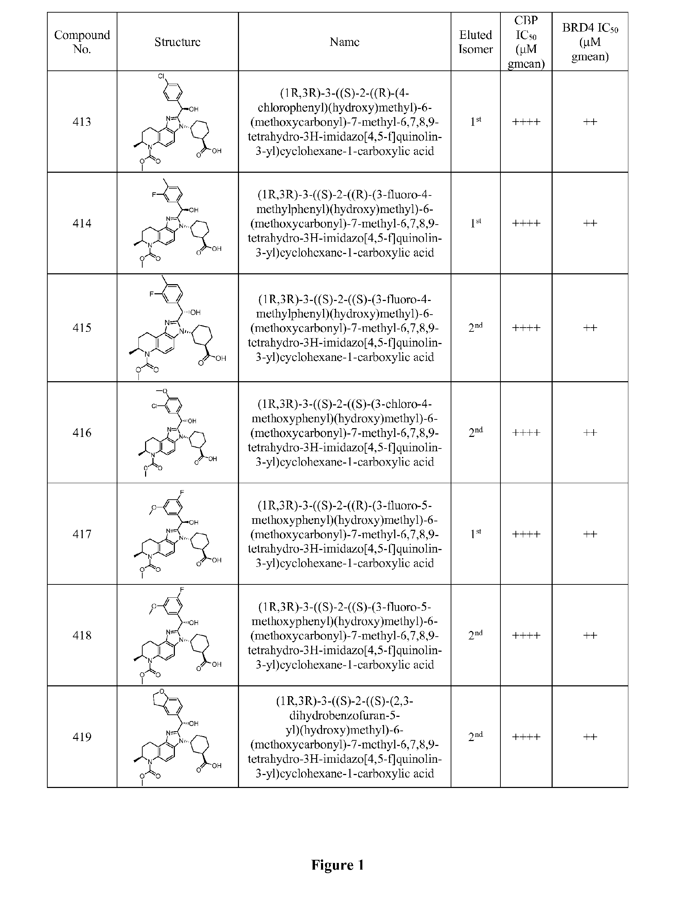

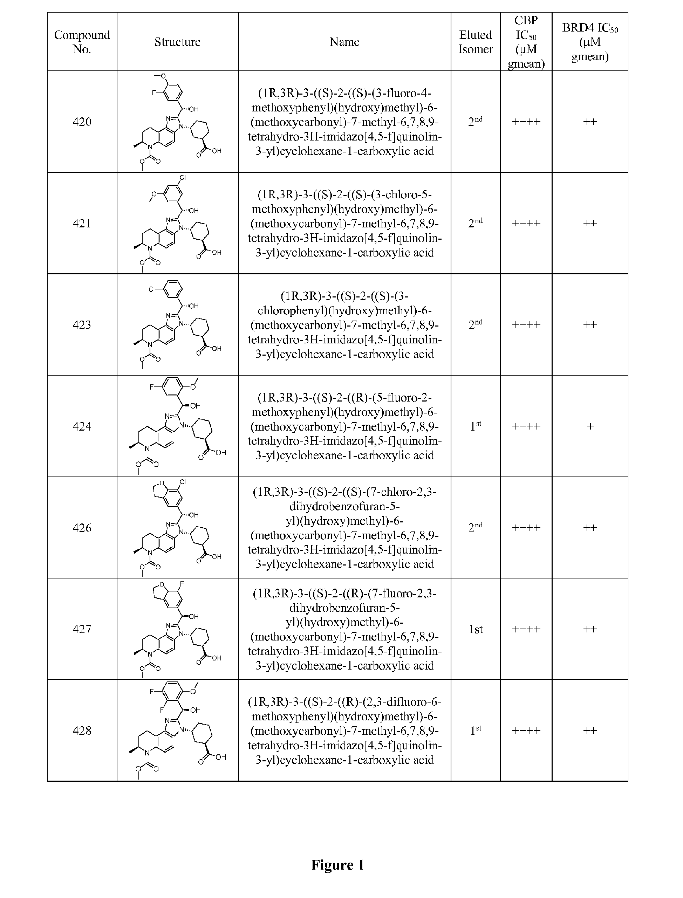

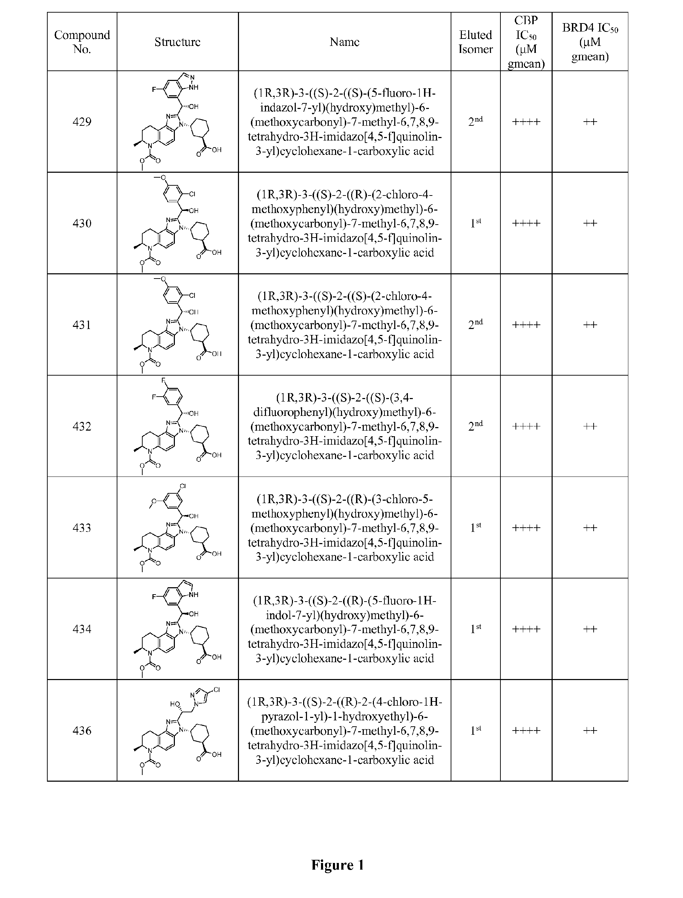

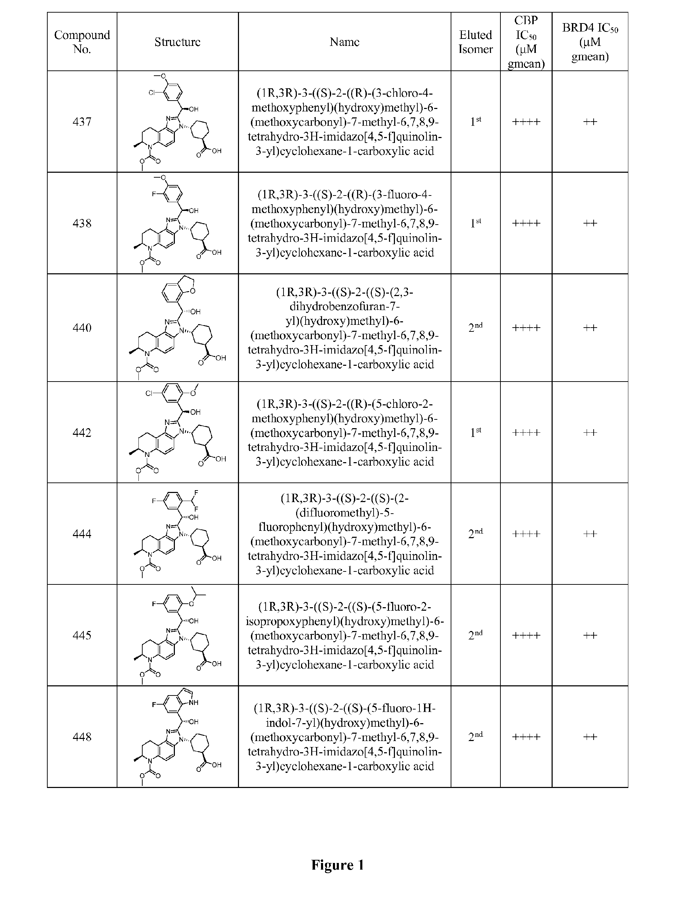

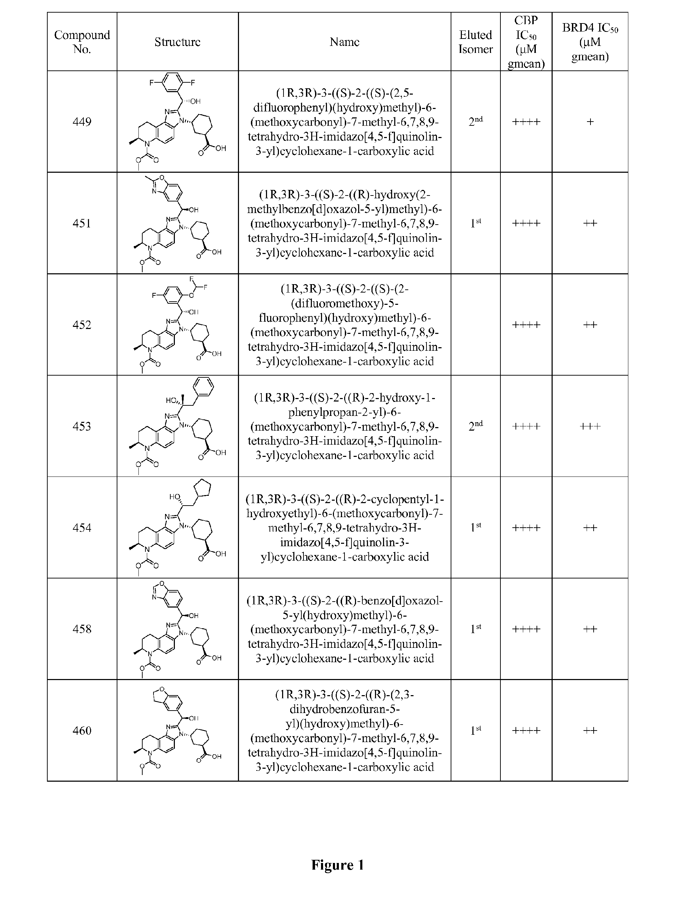

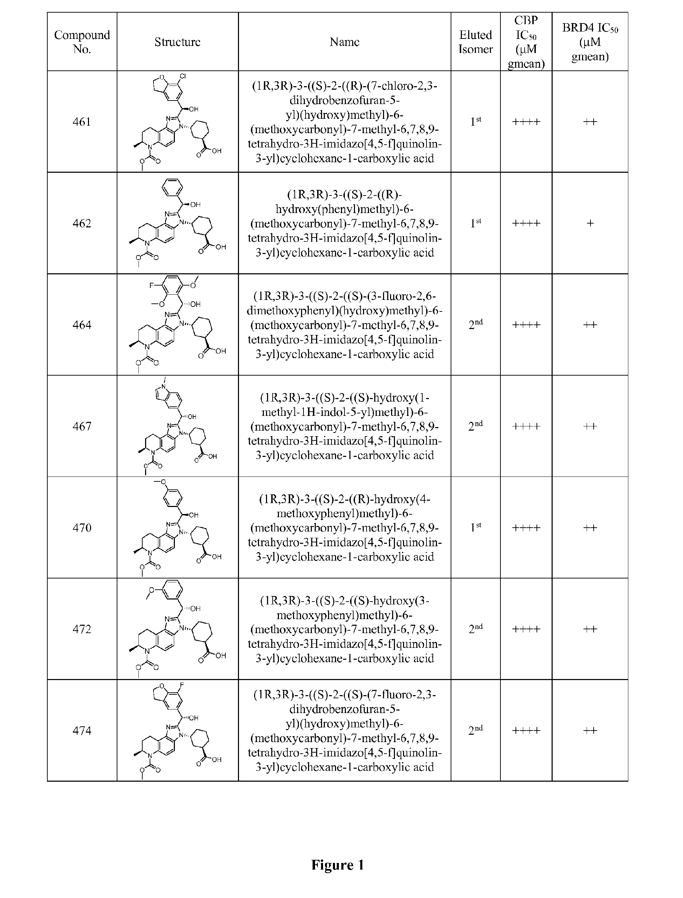

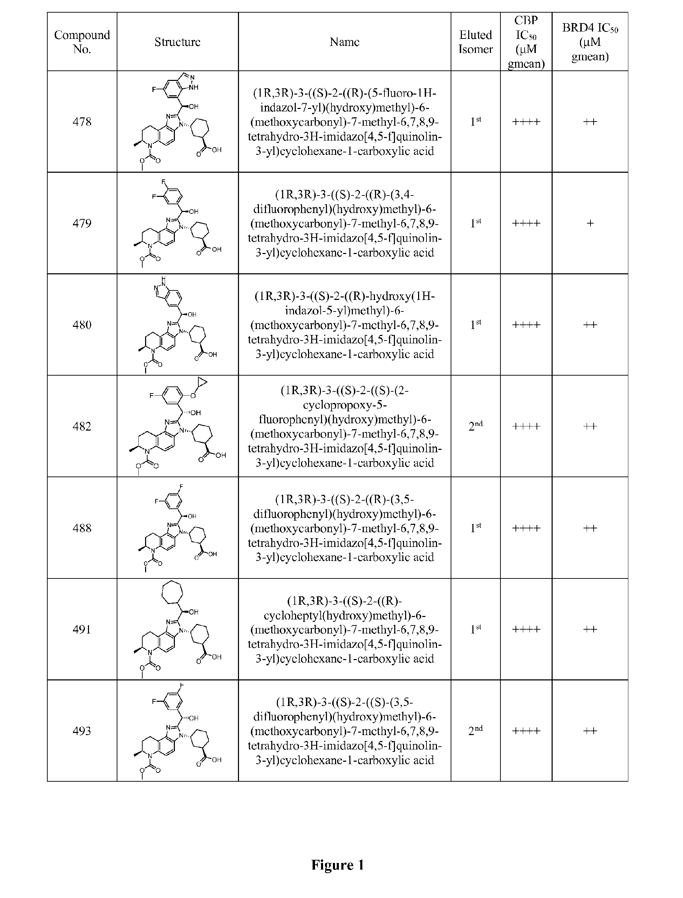

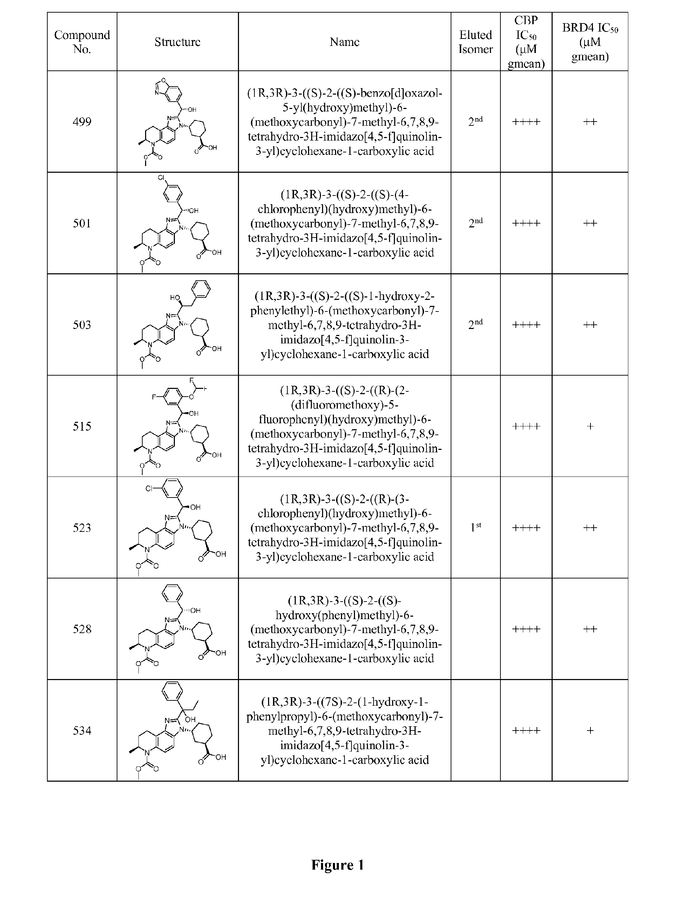

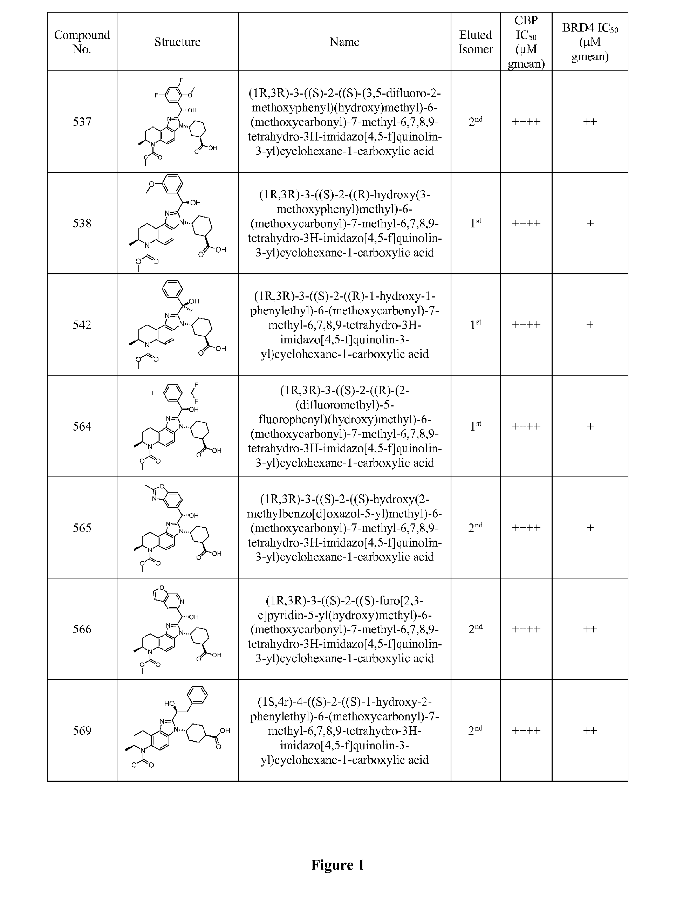

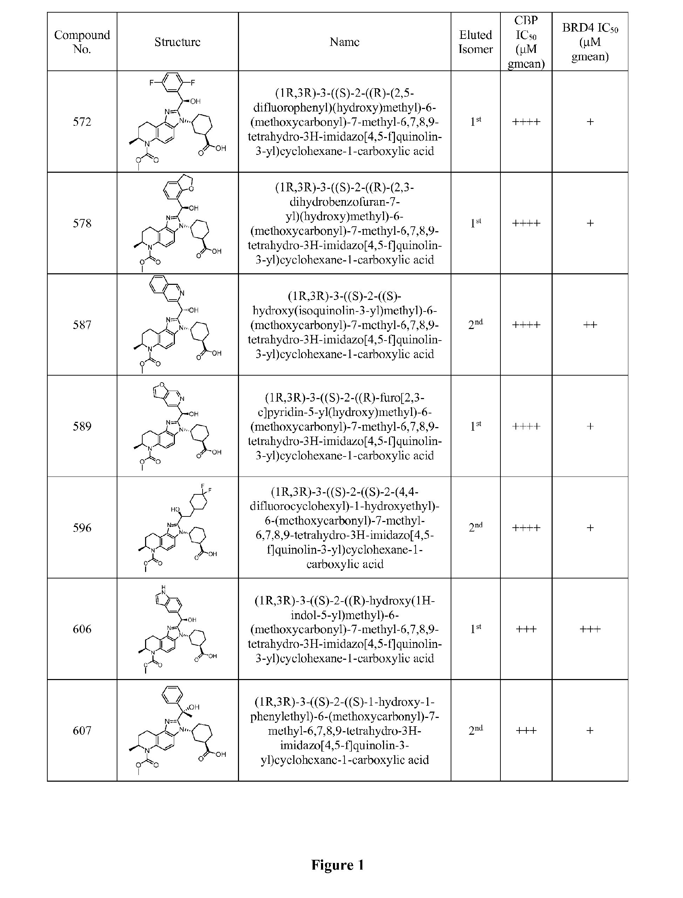

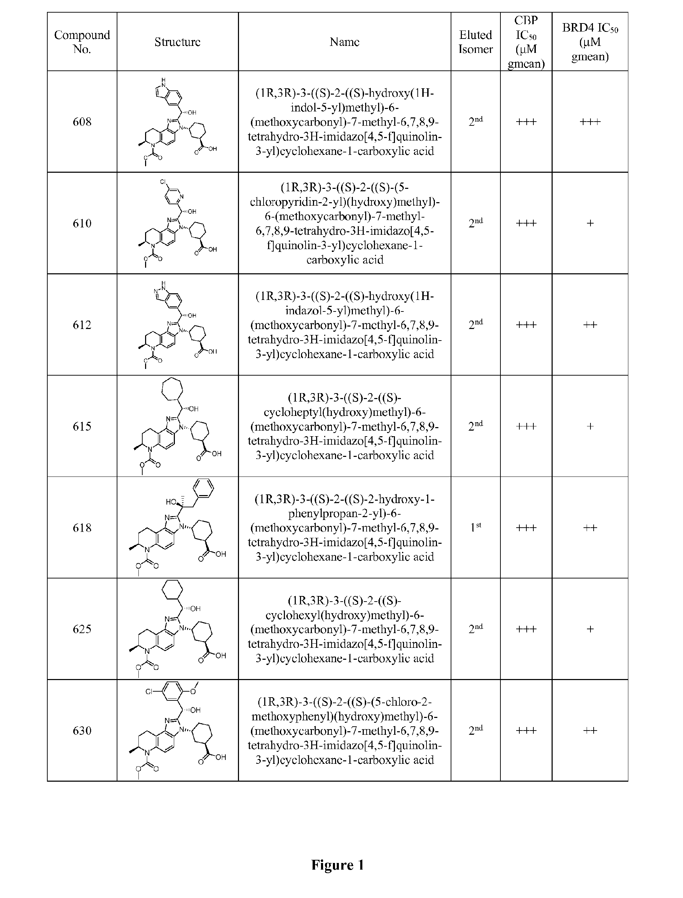

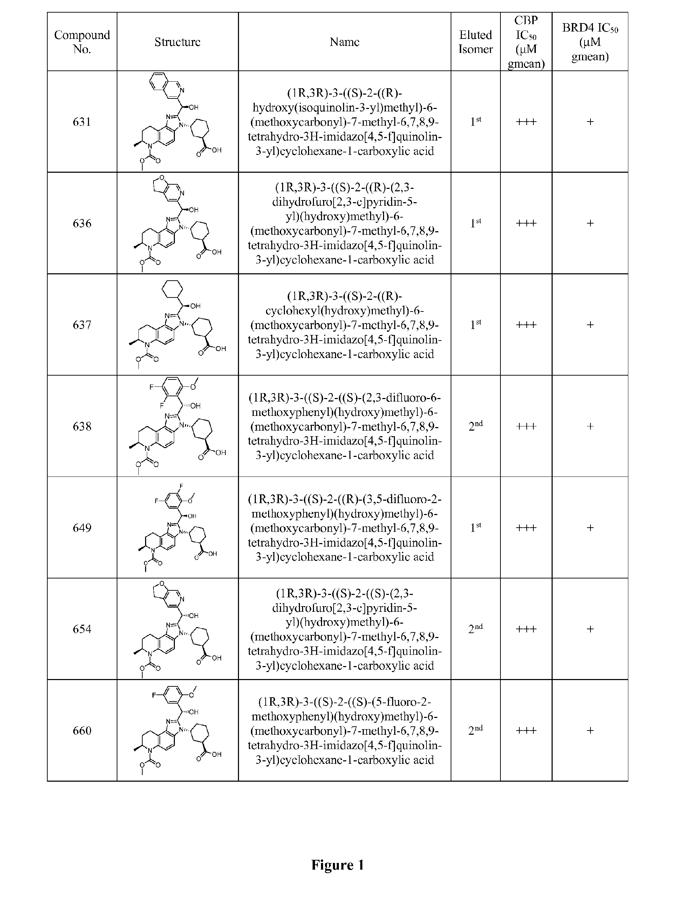

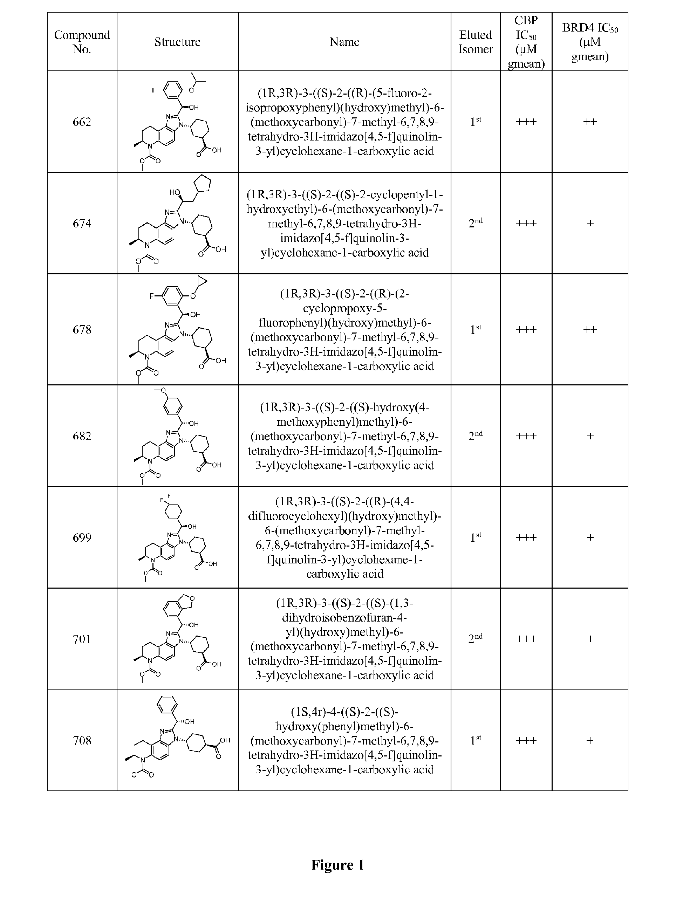

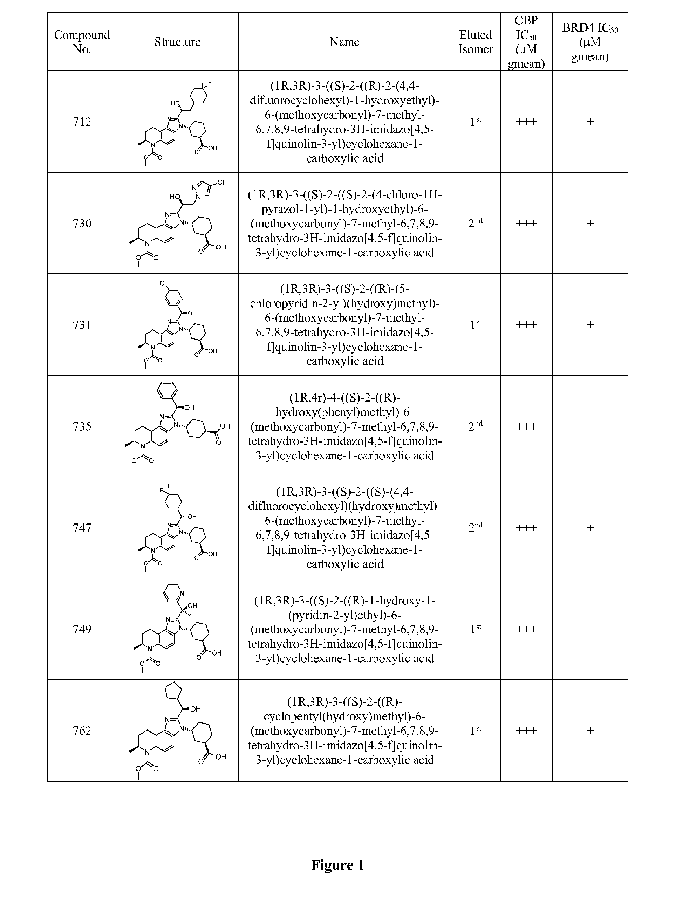

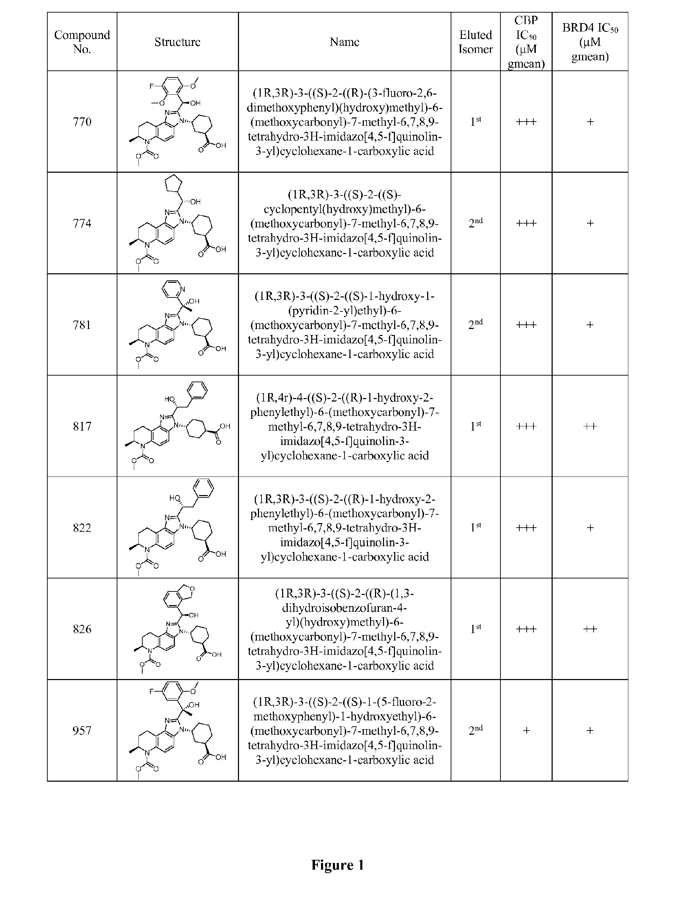

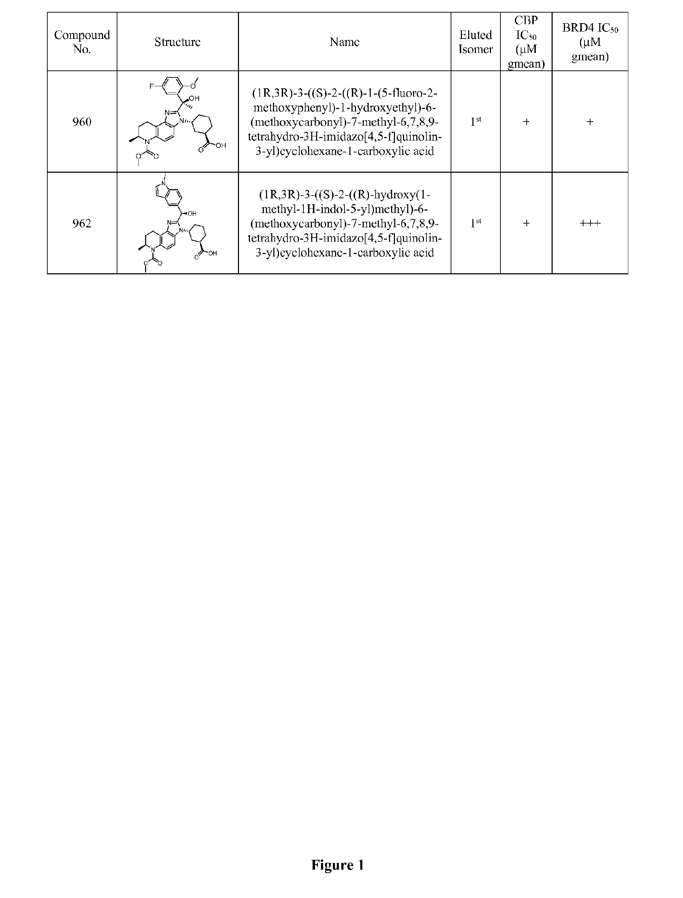

Figure 1 is a table of compounds in accordance with various embodiments of the disclosure.

### Detailed Description

The present disclosure relates to CBP Inhibitor Compounds, defined herein as compounds having one or more of the following characteristics when tested according to the HTRF biochemical Assay Protocol below in Example 5: (1) a CBP IC₅₀ value of less than 1 µM; and (2) a CBP IC₅₀ value of between 0.001 and 1 µM.

### Compounds of the Disclosure

One aspect of the present disclosure describes compounds of Formula (I): 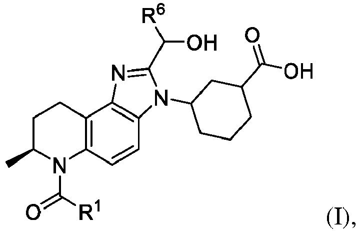 and pharmaceutically acceptable salts, enantiomers, hydrates, solvates, prodrugs, isomers, and tautomers thereof, wherein R¹ and R⁶ are described above.

In some examples, the compound of Formula (I) is a stereoisomer or enantiomer of Formula (I) selected from the group consisting of Formula (I-a), (I-b), (I-c), (I-d), (I-e), (I-f), (I-g), (I-h), (I-i), (I-j), (I-k), (I-l), (I-m), (I-n), (I-o) and (I-p):

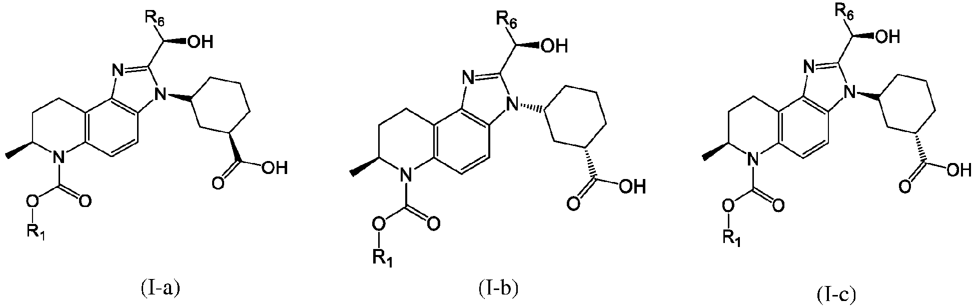

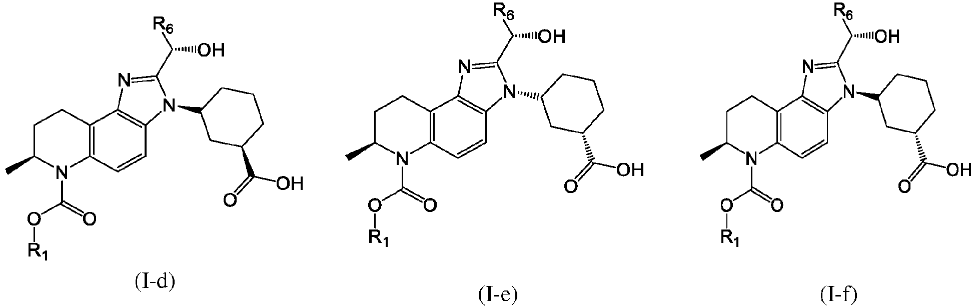

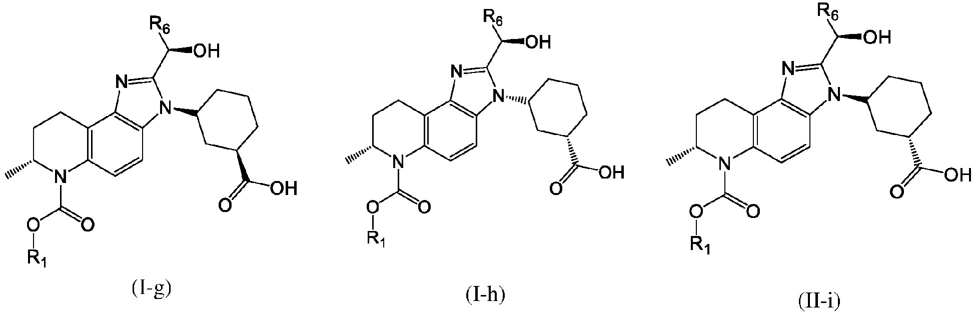

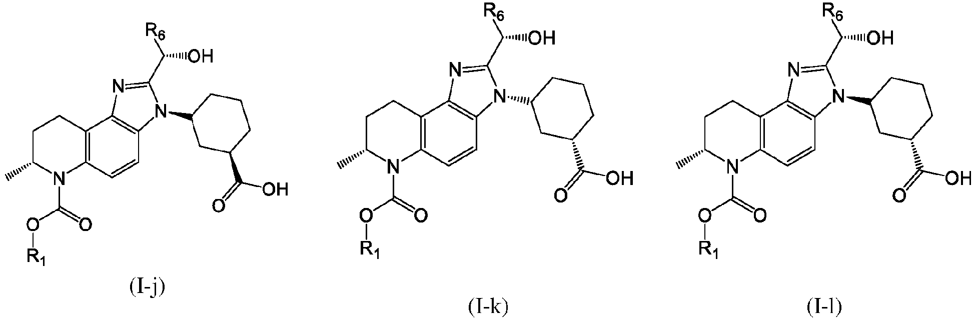

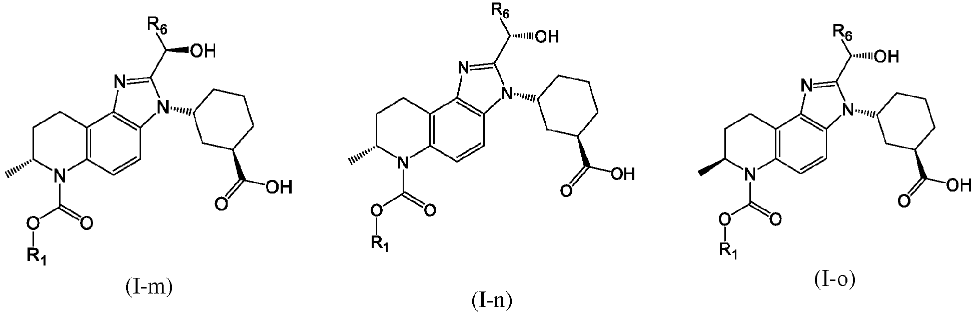

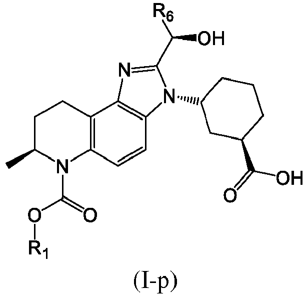

A compound of Formula (I) can be a stereoisomer thereof (e.g., a compound of Formula (I-a), (I-b), (I-c), (I-d), (I-e), (I-f), (I-g), (I-h), (I-i), (I-j), (I-k), (I-l), (I-m), (I-n), or (I-o) wherein R₁ is methyl and R₆ is phenyl optionally substituted with one or more R¹⁰).

In some preferred embodiments, the compound of Formula (I) is a compound of Formula (IV), including stereoisomers thereof: 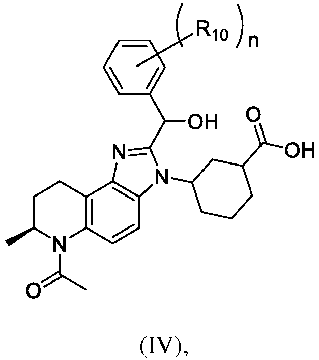 or a pharmaceutically acceptable salt thereof, wherein n is an integer of 0, 1, 2, 3, 4, or 5 (preferably, 0, 1 or 2) and R₁₀ is as defined above. Preferably, the compound of Formula (IV) is a compound of Formula (IV-a) (including, for example, compounds of Formula (IV-b), Formula (IV-c) or mixtures thereof), or pharmaceutically acceptable salts thereof, wherein n is an integer of 0, 1, 2, 3, 4 or 5 (preferably 0, 1 or 2) and R₁₀ is as defined above.

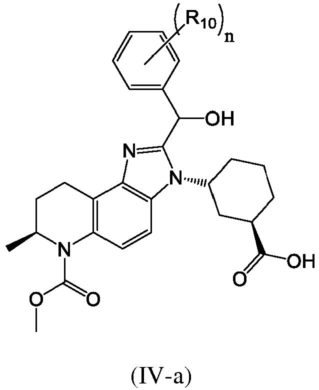

| | |
|---|---|
| 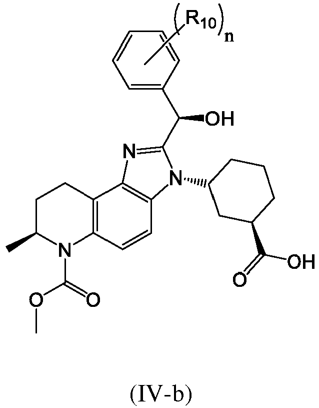 | 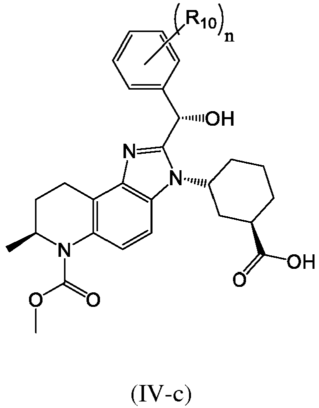 |

In certain preferred compounds of Formula (IV-a) including compounds of Formula (IV-b) and compounds of Formula (IV-c) wherein n is 0, 1, 2, 3, 4 or 5 and each R₁₀ is independently halogen or -OC₁-C₆alkyl, and wherein the -OC₁-C₆alkyl is optionally substituted with one or more halogen. For example, in certain compounds of Formula (IV-a), n is 0, 1 or 2 and each R₁₀ is independently halogen, or -OC₁alkyl optionally substituted with one or more halogen (e.g., fluorine or chlorine). In some compounds of Formula (IV-a), n is 2 and each R₁₀ is independently halogen (e.g., fluorine or chlorine), OC₁alkyl substituted with 1, 2 or 3 halogen (e.g., fluorine or chlorine), or methoxy.

Another aspect of the present disclosure is the provision of pharmaceutical compositions comprising therapeutically effective amounts of at least one compound of Formula (I). An aspect of the present disclosure concerns compounds which are, or can be, inhibitors of one or more bromodomains of the CBP/p300 family (e.g., compounds of Formula (I)).

In some embodiments, compounds of the disclosure have the Formula (I) 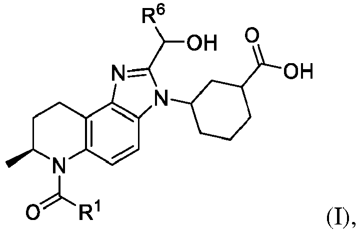 or a pharmaceutically acceptable salt, enantiomer, hydrate, solvate, isomer, or tautomer thereof, wherein:
R¹ is -C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OR⁵, -N(R⁵)₂, or -NHR⁵;
R⁵ is -C₁-C₆alkyl, -C₃-C₈cycloalkyl, heterocyclyl, aryl, or heteroaryl;
R⁶ is -C₁-C₆alkyl, -C₃-C₈cycloalkyl, -C₄-C₈cycloalkenyl, heterocyclyl, aryl, spirocycloalkyl, spiroheterocyclyl, heteroaryl, halogen, oxo, -(CH₂)ₙ-OR⁸,-C(O)R^{8'}, -C(O)OR⁸, or -C(O)NR⁸R⁹, wherein each alkyl, cycloalkyl, heterocyclyl, spirocycloalkyl, spiroheterocyclyl, heteroaryl, or aryl is optionally substituted with one or more R¹⁰;
R⁸ and R⁹ are each independently, at each occurrence, -H, -C₁-C₆alkyl, -C₂-C₆alkenyl, -C₂-C₆alkynyl, -C₃-C₈cycloalkyl, -C₄-C₈cycloalkenyl, heterocyclyl, aryl, heteroaryl, wherein each alkyl, alkenyl, alkynyl, cycloalkyl, cycloalkenyl, heterocyclyl, aryl, or heteroaryl is optionally substituted with one or more R¹⁰ or R¹¹; or
R⁸ and R⁹ may combine with the atom to which they are both attached to form a spiroheterocyclyl, heterocyclyl, or heteroaryl, wherein the formed spiroheterocyclyl, heterocyclyl,or heteroaryl is optionally substituted with one or more R¹⁰ or R¹¹;
R^{8'} is each independently, at each occurrence, -C₁-C₆alkyl, -C₂-C₆alkenyl, -C₂-C₆alkynyl, -C₃-C₈cycloalkyl, -C₄-C₈cycloalkenyl, heterocyclyl, aryl, heteroaryl, wherein each alkyl, alkenyl, alkynyl, cycloalkyl, cycloalkenyl, heterocyclyl, aryl, or heteroaryl is optionally substituted with one or more R¹⁰ or R¹¹; or
R¹⁰ is each independently, at each occurrence,-C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -OC₃-C₆cycloalkyl, -Oaryl, -Oheteroaryl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl), wherein each alkyl, alkenyl, alkynyl, cycloalkyl, cycloalkenyl, heterocyclyl, heteroaryl, or aryl is optionally substituted with one or more -R¹²;
wherein any two R¹⁰ when on non-adjacent atoms, can combine to form a bridging cycloalkyl or heterocyclyl;
wherein any two R¹⁰ when on adjacent atoms, can combine to form a cycloalkyl, heterocyclyl, aryl or heteroaryl; and
R¹² is independently, at each occurrence,-C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl).

In some embodiments, compounds of the disclosure have the Formula (I) 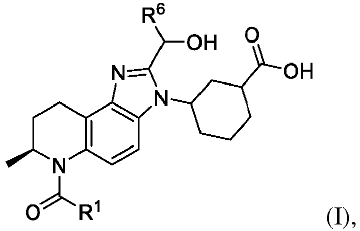 or a pharmaceutically acceptable salt, enantiomer, hydrate, solvate, isomer, or tautomer thereof, wherein:
R¹ is -OR⁵;
R⁵ is -C₁-C₆alkyl;
R⁶ is phenyl optionally substituted with one or more R¹⁰;
R¹⁰ is each independently, at each occurrence,-C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, -NO₂, -CN, -NH₂, -OC₁-C₆alkyl, -OC₃-C₆cycloalkyl, -Oaryl, -Oheteroaryl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl), wherein each alkyl, alkenyl, alkynyl, cycloalkyl, cycloalkenyl, heterocyclyl, heteroaryl, or aryl is optionally substituted with one or more -R¹²;
wherein any two R¹⁰ when on non-adjacent atoms, can combine to form a bridging cycloalkyl or heterocyclyl;
wherein any two R¹⁰ when on adjacent atoms, can combine to form a cycloalkyl, heterocyclyl, aryl or heteroaryl; and
R¹² is independently, at each occurrence,-C₁-C₆alkyl, -C2-C6alkenyl, -C2-C6alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl).

In some embodiments, compounds of the disclosure have the Formula (I) 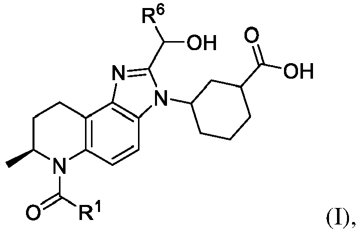 or a pharmaceutically acceptable salt, enantiomer, hydrate, solvate, isomer, or tautomer thereof, wherein:
R¹ is -OR⁵;
R⁵ is -C₁-C₃alkyl;
R⁶ is phenyl optionally substituted with one or more R¹⁰;
R¹⁰ is each independently, at each occurrence halogen, -OC₁-C₆alkyl, -OC₃-C₆cycloalkyl, -Oaryl, or -Oheteroaryl, wherein each alkyl, cycloalkyl, aryl or heteroaryl is optionally substituted with one or more -R¹²;
R¹² is halogen.

In some embodiments, compounds of the disclosure have the Formula (III): 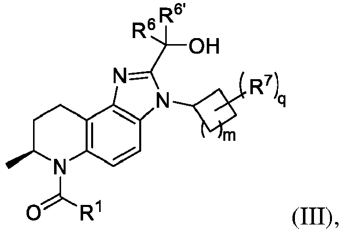 or a pharmaceutically acceptable salt thereof, wherein,
R¹ is -OR⁵;
R⁵ is -C₁-C₆alkyl, -C₃-C₈cycloalkyl, heterocyclyl, aryl, or heteroaryl;
R⁶ is -C₃-C₈cycloalkyl, -C₄-C₈cycloalkenyl, heterocyclyl, heteroaryl, aryl, wherein each cycloalkyl, cycloalkenyl, heterocyclyl, heteroaryl, or aryl is optionally substituted with one or more -R¹⁰;
R^{6'} is -H or -C₁-C₆alkyl;
R⁷ is -H, halogen, -OH, -CN, -OC₁-C₆alkyl, -NH₂, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -S(O)₂OH, -C(O)C₁-C₆alkyl, -C(O)NH₂, -C(O)NH(C₁-C₆alkyl), -C(O)N(C₁-C₆alkyl)₂, -C(O)OH, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, -S(O)₂NH₂, -N(C₁-C₆alkyl)S(O)(C₁-C₆alkyl) or tetrazole;
R¹⁰ is independently, at each occurrence, -C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -OC₃-C₆cycloalkyl, -Oaryl, -Oheteroaryl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -NHC(O)C₁-C₆alkyl, -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl), wherein each alkyl, alkenyl, alkynyl, cycloalkyl, cycloalkenyl, heterocyclyl, heteroaryl, or aryl is optionally substituted with one or more -R¹²;
R¹² is independently, at each occurrence, -C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl);
m is an integer from 0 to 5; and
q is an integer from 0 to 4.

Multiple embodiments of the compounds of Formula (III) are provided herein. In some embodiments R¹² is halogen. In some embodiments m is 3. In some embodiments R^{6'} is H. In some embodiments R⁶ is aryl. In some embodiments R⁷ is -C(O)OH. In some embodiments R⁵ is methyl.

In some embodiments, compounds of the disclosure have the Formula (I): 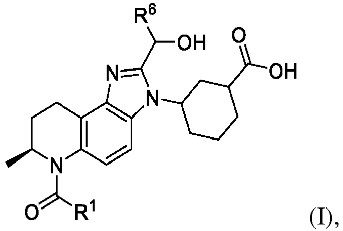 or a pharmaceutically acceptable salt thereof, wherein:
R¹ is -OR⁵;
R⁵ is -C₁-C₆alkyl; and
R⁶ is phenyl optionally substituted with one or more R¹⁰;
R¹⁰ is independently, at each occurrence, -C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, -NO₂, -CN, -NH₂, -OC₁-C₆alkyl, -OC₃-C₆cycloalkyl, -Oaryl, -Oheteroaryl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -NHC(O)C₁-C₆alkyl, -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl), wherein each alkyl, alkenyl, alkynyl, cycloalkyl, cycloalkenyl, heterocyclyl, heteroaryl, or aryl is optionally substituted with one or more -R¹²; and
R¹² is independently, at each occurrence, -C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl).

In some embodiments, compounds of the disclosure have the Formula (I): 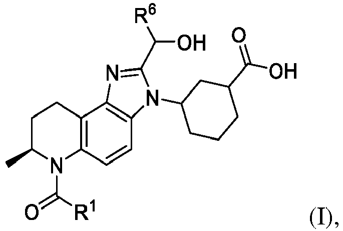 or a pharmaceutically acceptable salt thereof, wherein:
R¹ is -OR⁵;
R⁵ is -C₁-C₃alkyl;
R⁶ is phenyl optionally substituted with one or more R¹⁰;
R¹⁰ is independently, at each occurrence halogen, -OC₁-C₆alkyl, -OC₃-C₆cycloalkyl, - Oaryl, or -Oheteroaryl, wherein each alkyl, cycloalkyl, aryl or heteroaryl is optionally substituted with one or more -R¹²; and
R¹² is halogen.

In some embodiments, R⁶ is aryl optionally substituted with one or more R¹⁰. In some embodiments, R⁶ is phenyl optionally substituted with one or more R¹⁰.

In some embodiments R⁵ is -C₁-C₃alkyl. In some embodiments, R⁵ is methyl.

In some embodiments, R¹⁰ is independently, at each occurrence, halogen or -OC₁-C₆alkyl, wherein -OC₁-C₆alkyl is optionally substituted with halogen.

In some embodiments, R₁ is -OR⁵.

In some embodiments, R¹ is -OR⁵, -N(R⁵)₂, -NHR⁵, or -C₁-C₆alkyl. In some embodiments, R¹ is -OR⁵. In some embodiments, R¹ is -OR⁵ or -C₁-C₆alkyl. In some embodiments, R⁵ of R¹ is -C₁-C₆alkyl. In some embodiments, R¹ is -OR⁵; and R⁵ is -C₁-C₆alkyl. In some embodiments R¹ is -OCH₃. In some embodiments, R¹ is -C₂-C₆alkenyl, -C₂-C₆alkynyl, -C₃-C₈cycloalkyl, -C₄-C₈cycloalkenyl, heterocyclyl, heteroaryl, or aryl. In some embodiments, R¹ is -C₁-C₆alkyl. In some embodiments, R¹ is methyl, ethyl or propyl. In some embodiments, R¹ is methyl. In some embodiments, R¹ is -C₂-C₆alkenyl. In some embodiments, R¹ is aryl.

In some embodiments, R⁵ is -C₁-C₆alkyl, -C₃-C₈cycloalkyl, heterocyclyl, aryl, or heteroaryl. In some embodiments, R⁵ is -C₁-C₆alkyl. In some embodiments, R⁵ is -C₁-C₃alkyl. In some embodiments, R⁵ is methyl. In some embodiments, R⁵ is ethyl.

In some embodiments, R⁶ is -C₁-C₆alkyl, -C₃-C₈cycloalkyl, -C₄-C₈cycloalkenyl, heterocyclyl, or aryl. In some embodiments, R⁶ is -C₁-C₆alkyl optionally substituted with one or more R¹⁰. In some embodiments, R⁶ is aryl optionally substituted with one or more R¹⁰. In some embodiments, R⁶ is heteroaryl optionally substituted with one or more R¹⁰. In some embodiments, R⁶ is -C(O)OH. In some embodiments, R⁶ is halogen. In some embodiments, R⁶ is -C₁-C₆alkyl, -C₃-C₈cycloalkyl, -C₄-C₈cycloalkenyl, heterocyclyl, aryl, spirocycloalkyl, spiroheterocyclyl, heteroaryl, halogen, oxo, -(CH₂)ₙ-OR⁸, -C(O)R^{8'}, -C(O)OR⁸, or -C(O)NR⁸R⁹, wherein each alkyl, cycloalkyl, heterocyclyl, spirocycloalkyl, spiroheterocyclyl, heteroaryl, or aryl is optionally substituted with one or more R¹⁰.

In some embodiments, R⁸ is -C₁-C₆alkyl, -C₂-C₆alkenyl, -C₂-C₆alkynyl, -C₃-C₈cycloalkyl, -C₄-C₈cycloalkenyl, heterocyclyl, aryl, heteroaryl, wherein each alkyl, alkenyl, alkynyl, cycloalkyl, cycloalkenyl, heterocyclyl, aryl, or heteroaryl is optionally substituted with one or more R¹⁰ or R¹¹. In some embodiments, R⁸ is -H. In some embodiments, R⁸ is -C₁-C₆alkyl, -C₃-C₈cycloalkyl, -C₄-C₈cycloalkenyl, heterocyclyl, or aryl, wherein R⁸ is optionally substituted with R¹⁰ or R¹¹. In some embodiments, R⁸ is -C₁-C₆alkyl optionally substituted with one or more R¹⁰ or R¹¹. In some embodiments, R⁸ is aryl optionally substituted with one or more R¹⁰ or R¹¹. In some embodiments, R⁸ is heteroaryl optionally substituted with one or more R¹⁰ or R¹¹.

In some embodiments, R^{8'} is -C₁-C₆alkyl, -C₂-C₆alkenyl, -C₂-C₆alkynyl, -C₃-C₈cycloalkyl, -C₄-C₈cycloalkenyl, heterocyclyl, aryl, heteroaryl, wherein each alkyl, alkenyl, alkynyl, cycloalkyl, cycloalkenyl, heterocyclyl, aryl, or heteroaryl is optionally substituted with one or more R¹⁰ or R¹¹. In some embodiments, R^{8'} is -C₁-C₆alkyl, -C₃-C₈cycloalkyl, -C₄-C₈cycloalkenyl, heterocyclyl, or aryl, wherein R^{8'} is optionally substituted with R¹⁰ or R¹¹. In some embodiments, R^{8'} is -C₁-C₆alkyl optionally substituted with one or more R¹⁰ or R¹¹. In some embodiments, R^{8'} is aryl optionally substituted with one or more R¹⁰ or R¹¹. In some embodiments, R^{8'} is heteroaryl optionally substituted with one or more R¹⁰ or R¹¹.

In some embodiments, R⁹ is -C₁-C₆alkyl, -C₂-C₆alkenyl, -C₂-C₆alkynyl, -C₃-C₈cycloalkyl, -C₄-C₈cycloalkenyl, heterocyclyl, aryl, heteroaryl, wherein each alkyl, alkenyl, alkynyl, cycloalkyl, cycloalkenyl, heterocyclyl, aryl, or heteroaryl is optionally substituted with one or more R¹⁰ or R¹¹. In some embodiments, R⁹ is -H. In some embodiments, R⁹ is -C₁-C₆alkyl, -C₃-C₈cycloalkyl, -C₄-C₈cycloalkenyl, heterocyclyl, or aryl, wherein R⁹ is optionally substituted with R¹⁰ or R¹¹. In some embodiments, R⁹ is -C₁-C₆alkyl optionally substituted with one or more R¹⁰ or R¹¹. In some embodiments, R⁹ is aryl optionally substituted with one or more R¹⁰ or R¹¹. In some embodiments, R⁹ is heteroaryl optionally substituted with one or more R¹⁰ or R¹¹.

In some embodiments, R¹⁰ is -C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl), wherein each alkyl, alkenyl, alkynyl, cycloalkyl, cycloalkenyl, heterocyclyl, heteroaryl, or aryl is optionally substituted with one or more -R¹². In some embodiments, R¹⁰ is -C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, wherein R¹⁰ is substituted with R¹². In some embodiments, R¹⁰ is halogen. In some embodiments, R¹⁰ is each independently, at each occurrence,-C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -OC₃-C₆cycloalkyl, Oaryl, Oheteroaryl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl), wherein each alkyl, alkenyl, alkynyl, cycloalkyl, cycloalkenyl, heterocyclyl, heteroaryl, or aryl is optionally substituted with one or more -R¹²; wherein any two R¹⁰ when on non-adjacent atoms, can combine to form a bridging cycloalkyl or heterocyclyl; wherein any two R¹⁰ when on adjacent atoms, can combine to form a cycloalkyl, heterocyclyl, aryl or heteroaryl. In some embodiments, R¹⁰ is each independently, at each occurrence halogen or -OC₁-C₆alkyl, -OC₃-C₆cycloalkyl, -Oaryl, -Oheteroaryl, wherein each alkyl, cycloalkyl, aryl or heteroaryl is optionally substituted with one or more -R¹².

In some embodiments, R¹¹ is -C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl), wherein each alkyl, alkenyl, alkynyl, cycloalkyl, cycloalkenyl, heterocyclyl, heteroaryl, or aryl is optionally substituted with one or more -R¹². In some embodiments, R¹¹ is -C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, wherein R¹¹ is substituted with R¹². In some embodiments, R¹¹ is halogen.

In some embodiments, R¹² is -C₁-C₆alkyl, -C₂-C₆alkenyl, -C₂-C₆alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl). In some embodiments, R¹² is -H. In some embodiments, R¹² is halogen.

In some embodiments, n is 1. In some embodiments, n is 2. In some embodiments, n is 3. In some embodiments, n is 4.

In some embodiments, R¹ is -C₁-C₆alkyl, -C₂-C₆alkenyl, -C₂-C₆alkynyl, -C₃-C₈cycloalkyl, -C₄-C₈cycloalkenyl, heterocycloalkyl, heteroaryl, or aryl.

Preferably, the compound is a CBP Inhibitor Compound of Formula (I) wherein R₁ is -OCH3. In some embodiments, a CBP Inhibitor Compound of Formula (I) includes R₁ is -OCH3 and R6 is C₆ aryl (phenyl) optionally substituted with one or more R₁₀. In some embodiments, a CBP Inhibitor Compound of Formula (I) includes R₁ is -OCH3 and R₆ is C6 aryl (phenyl) optionally substituted with one or more R₁₀ being selected from the group consisting of halogen (e.g., fluorine) and methoxy, wherein the methoxy is optionally substituted with one or more R₁₂. In some embodiments, a CBP Inhibitor Compound of Formula (I) includes R₁ is -OCH3 and R₆ is C6 aryl (phenyl) optionally substituted with one or more R₁₀ being selected from the group consisting of halogen (e.g., fluorine) and methoxy, wherein the methoxy is optionally substituted with one or more R₁₂ and R₁₂ is a halogen (preferably, fluorine).

In some embodiments, a compound of the disclosure is a compound selected from Figure 1, or a pharmaceutically acceptable salt thereof.

### Method of Synthesizing the Compounds

The compounds of the present disclosure may be made by a variety of methods, including standard chemistry. Suitable synthetic routes are depicted in the examples given below.

The compounds of the present disclosure, *i.e*., compounds of Formula (I), or a pharmaceutically acceptable salt, enantiomer, hydrate, solvate, prodrug, isomer, or tautomer thereof, may be prepared by methods known in the art of organic synthesis as set forth in part by the following synthetic schemes. In the schemes described below, it is well understood that protecting groups for sensitive or reactive groups are employed where necessary in accordance with general principles or chemistry. Protecting groups are manipulated according to standard methods of organic synthesis (T. W. Greene and P. G. M. Wuts, "Protective Groups in Organic Synthesis", Third edition, Wiley, New York 1999). These groups are removed at a convenient stage of the compound synthesis using methods that are readily apparent to those skilled in the art. The selection processes, as well as the reaction conditions and order of their execution, shall be consistent with the preparation of compounds of Formula (I).

Those skilled in the art will recognize if a stereocenter exists in the compounds of Formula (I). Accordingly, the present disclosure includes both possible stereoisomers (unless specified in the synthesis) and includes not only racemic compounds but the individual enantiomers and/or diastereomers as well. When a compound is desired as a single enantiomer or diastereomer, it may be obtained by stereospecific synthesis or by resolution of the final product or any convenient intermediate. Resolution of the final product, an intermediate, or a starting material may be affected by any suitable method known in the art. See, for example, "Stereochemistry of Organic Compounds" by E. L. Eliel, S. H. Wilen, and L. N. Mander (Wiley-lnterscience, 1994).

The compounds described herein may be made from commercially available starting materials or synthesized using known organic, inorganic, and/or enzymatic processes.

The disclosure also includes pharmaceutical compositions comprising one or more CBP Inhibitor Compounds as described herein, or a pharmaceutically acceptable salt thereof, and a pharmaceutically acceptable excipient. In some embodiments, pharmaceutical compositions reported herein can be provided in a unit dosage form (e.g., capsule, tablet or the like). Pharmaceutical compositions comprising a compound of Formula (I) can be provided in an oral dosage form such as a capsule or tablet. The oral dosage form optionally comprises one or more fillers, disintigrants, lubricants, glidants, anti-adherents and/or anti-statics. In some embodiments, an oral dosage form is prepared via dry blending. In some embodiments, an oral dosage form is a tablet and is prepared via dry granulation. For example, a CBP Inhibitor compound of the present disclosure can be dosed at 1 mg to 1 g at a therapeutically effective frequency.The pharmaceutical compositions may be orally administered in any orally acceptable dosage form. Accordingly, a patient and/or subject can be selected for treatment using a compound described herein by first evaluating the patient and/or subject to determine whether the subject is in need of inhibition of CBP, and if the subject is determined to be in need of inhibition of CBP, then administering to the subject a composition described herein.

A pharmaceutical composition can comprise one or more compounds of Formula (I) including any compound disclosed in the examples below, as provided herein. In one example, an active pharmaceutical ingredient (API) can comprise about 90% or more of a compound of Formula (I) and up to about 10% (preferably up to about 5%, most preferably up to about 2.5% including about 1.5%) of the compound of Formula (I). Oral dosage forms comprising a compound of Formula (I) can be prepared as a drug-in-capsule (DiC), encapsulated simple dry-blend granulation, and lipid-based solution in hard shell capsule. The capsules can contain pharmaceutically acceptable excipients, and encapsulated capsules can be packaged in high-density polyethylene induction sealed bottles.

### EXAMPLES

### Definitions used in the following Schemes and elsewhere herein are:

- ACN: acetonitrile
- Ac₂O: acetic anhydride
- (±)BINAP: (±)-2,2'-Bis(diphenylphosphino)-1,1'-binaphthalen
- Boc: tert-butoxycarbonyl
- n-BuOH: butanol
- cm: centimeter
- DCE: 1,2-dichloroethane
- DCM: dichloromethane or methylene chloride
- DEA: diethylamine
- DMC: 2-Chloro-4,5-dihydro-1,3-dimethyl-1*H*-imidazolium chloride
- DMP: Dess-Martin periodinane
- DMTMM: 4-(4,6-Dimethoxy-1,3,5-triazin-2-yl)-4-methylmorpholinium chloride
- DIEA: N,N-diisopropylethylamine
- DMAP: 4-(dimethylamino)pyridine
- DMF: N,N-dimethylformamide
- DMSO: dimethylsulfoxide
- DPPA: diphenylphosphoryl azide
- dppf: bis(diphenylphosphino)ferrocene
- ES: electrospray ionization
- Et₃N: triethylamine
- EtOAc: ethyl acetate
- EtOH: ethanol
- FA: formic acid
- FCC: flash column chromatography
- h: hours
- HATU: 2-(3H-[1,2,3]triazolo[4,5-b]pyridin-3-yl)-1,1,3,3-tetramethylisouronium hexafluorophosphate
- HCl: hydrogen chloride
- HOAc: acetic acid
- HPLC: high performance liquid chromatography
- (*i*-Pr)₂NEt: N,N-diisopropylethylamine
- L: liter
- LC/MS: liquid chromatography/mass spectrometry
- LDA: lithium diisopropylamine
- K₂CO₃: potassium carbonate
- MeOH: methanol
- mL: milliliter
- mmol: millimole
- mg: milligram
- MHz: megahertz
- MS: mass spectrometry
- m/z: mass/charge ratio
- NBS: N-bromosuccinimide
- nm: nanometer
- NMM: 4-methylmorpholine
- NMR: nuclear magnetic resonance
- Pd₂(dba)₃: tris(dibenzylideneacetone)dipalladium
- Ph₃P: triphenylphosphine
- PhCHO: benzaldehyde
- PhMe: toluene
- ppm: parts per million
- rt: room temperature
- RT: rentention time
- SFC: supercritical fluid chromatography
- STAB: sodium triacetoxyborohydride
- *p*-TSA: para-toluenesulfonic anhydride
- *p*-TsOH: para-toluenesulfonic acid
- TFA: trifluoroacetic acid
- TFAA: trifluoroacetic anhydride
- THF: tetrahydrofuran
- UV: ultraviolet
- XPhos: 2-dicyclohexylphosphino-2',4',6'-triisopropylbiphenyl

### Materials

Unless otherwise noted, all materials were obtained from commercial suppliers and were used without further purification. Anhydrous solvents were obtained from Sigma-Aldrich (Milwaukee, WI) and used directly. All reactions involving air- or moisture-sensitive reagents were performed under a nitrogen atmosphere and all reactions utilizing microwave irraditation were run on a Biotage Initiator EXP EU instrument.

Unless otherwise noted, mass-triggered HPLC purification and/or purity and low resolution mass spectral data were measured using either: (1) Waters Acquity ultra performance liquid chromatography (UPLC) system (Waters Acquity UPLC with Sample Organizer and Waters Micromass ZQ Mass Spectrometer) with UV detection at 220 nm and a low resonance electrospray positive ion mode (ESI) (Column: Acquity UPLC BEH C18 1.7µm 2.1 X 50 mm; gradient: 5-100% Solvent B (95/5/0.09%: Acetonitrile/Water/Formic Acid) in Solvent A (95/5/0.1%: 10mM Ammonium Formate/Acetonitrile/Formic Acid) for 2.2 min then 100-5% Solvent B in Solvent A for 0.01 min then hold at 5% Solvent B in Solvent A for 0.29 min) or (2) Waters HT2790 Alliance high performance liquid chromatography (HPLC) system (Waters 996 PDA and Waters ZQ Single Quad Mass Spectrometer) with UV detection at 220 nm and 254 nm and a low resonance electrospray ionization (positive/negative) mode (ESI) (Column: XBridge Phenyl or C18, 5 µm 4.6x50 mm; gradient: 5-95% Solvent B (95% methanol/5% water with 0.1% Formic Acid) in Solvent A (95% water/5% methanol with 0.1% Formic Acid) for 2.5 min then hold at 95% Solvent B in Solvent A for 1 min (purity and low resolution MS only).

### General Methods of Compound Preparation

Described herein are methods of synthesizing the compounds of the present disclosure. Compounds of the present disclosure can be synthesized according to the synthetic schemes provided below. Preparation of the starting material for Schemes 1 and 2 ("Intermediate 1") is described below. Preparation of the starting material for Schemes 3 and 4 can be found in Example 1, Part A of U.S. Patent No. 4,404,207.

Unless otherwise specified, the substituents R² and R³ in the following reaction schemes are defined as follows, and R⁶ is as defined in the description and claims.

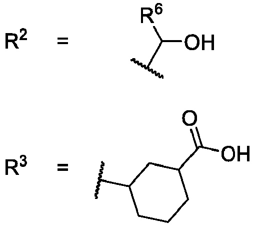

Scheme 1 provides methods useful for synthesizing compounds of Formula I.

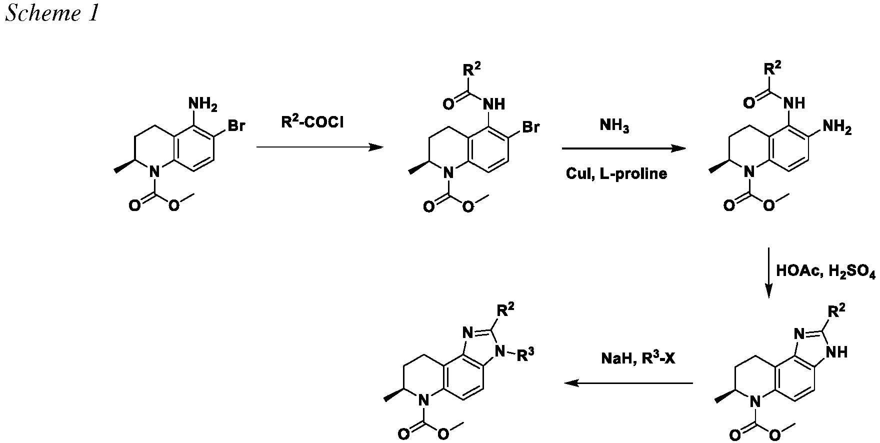

Scheme 2 provides methods useful for synthesizing compounds of Formula I.

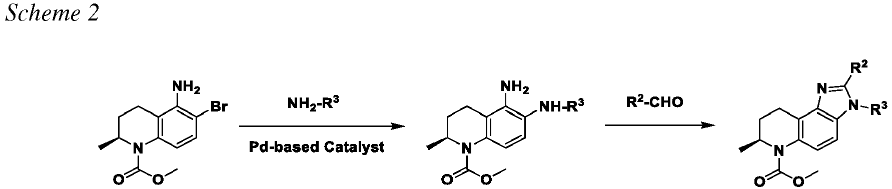

Alternatively, Scheme 3 provides methods useful for synthesizing certain compounds of Formula I.

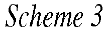

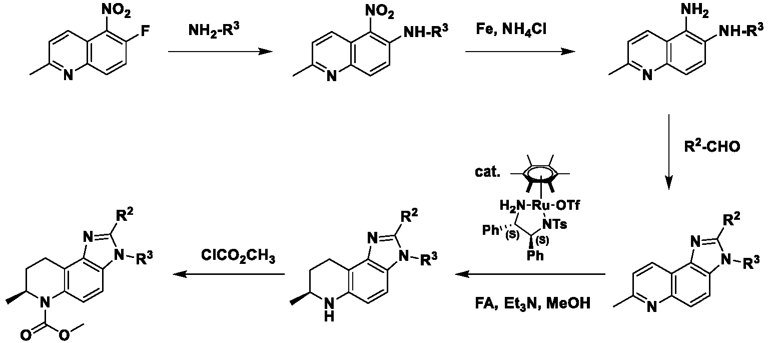

Alternatively, Scheme 4 provides methods useful for synthesizing certain compounds of Formula I.

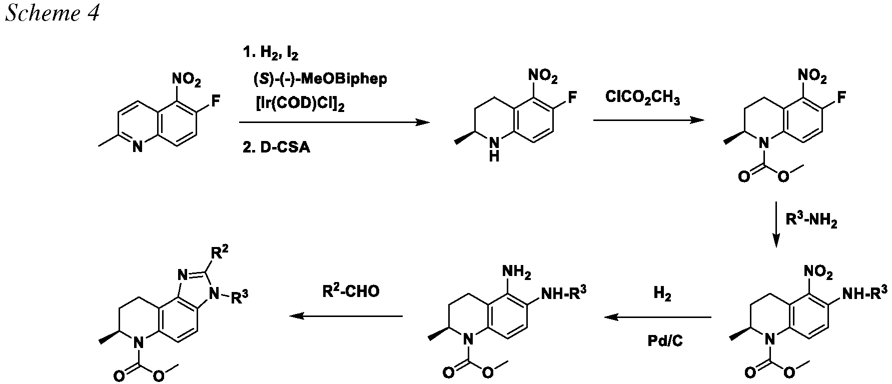

### Preparation of Intermediate 1: methyl (S)-5-amino-6-bromo-2-methyl-3,4-dihydroquinoline-1(2H)-carboxylate

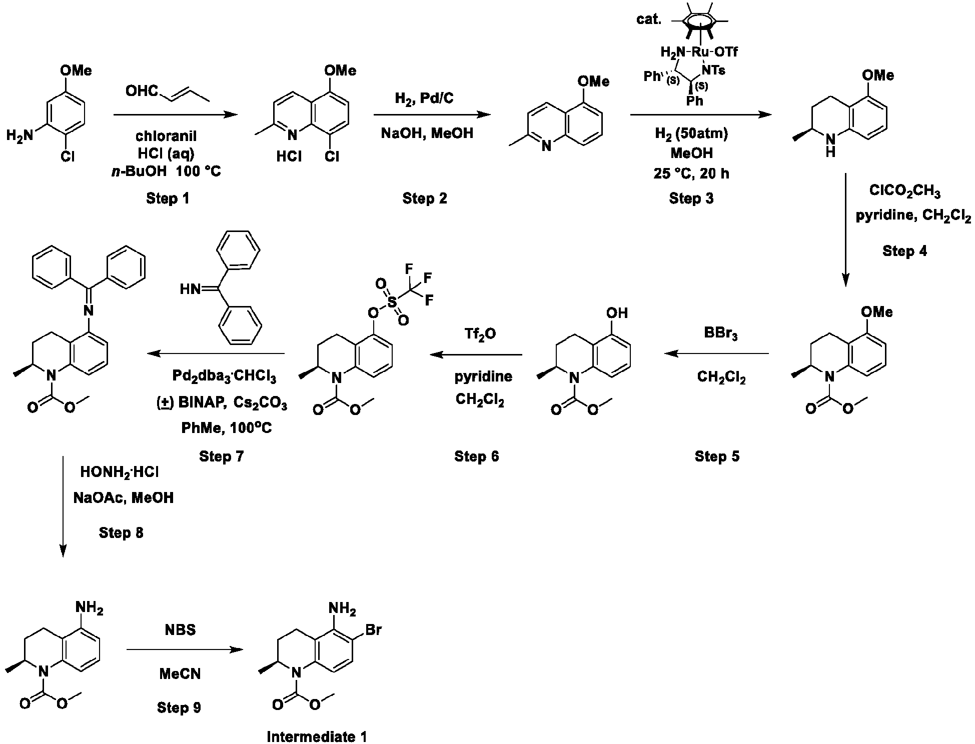

### Step 1. 8-chloro-5-methoxy-2-methylquinoline hydrochloride

Into a 5L 4-necked round-bottom flask purged and maintained with an inert atmosphere of nitrogen, 2-chloro-5-methoxyaniline (250 g, 1.59 mol) was dissolved in 1-butanol (1200 mL). Then hydrochloric acid (aq, 36.5%, 526.5 mL) and chloranil (456.5 g, 1.86 mol) were added. The resulting mixture was stirred for 1 h at 100°C under nitrogen atmosphere. Then a solution of (E)-but-2-enal (169 mL, 2.06 mol) in 1-butanol (300 mL) was added dropwise. The resulting solution was stirred for 1 h at 100°C under nitrogen atmosphere. The oil bath was cooled to 70°C and tetrahydrofuran (1500mL) was added. Then the resulting mixture was stirred for 1 h at 70°C. The reaction mixture was cooled to 0°C and the solids were filtered. The solids were washed with tetrahydrofuran (3L) at 0°C. This afforded the title compound (300g, 77%) as a yellow solid. MS: (ES, *m*/*z*): 208, 210 [M+H]⁺. then dried in an oven to afford 8-chloro-5-methoxy-2-methylquinoline hydrochloride (83.0 g, 74%) as a yellow solid. MS (ES, *m*/*z*): 208 [M+H]⁺.

### Step 2. 5-methoxy-2-methylquinoline

Into a 1000-mL 3-necked round-bottom flask, 8-chloro-5-methoxy-2-methylquinoline hydrochloride (50 g, 204.82 mmol) was dissolved in methanol (300 mL). Then sodium hydroxide (3M, 205 mL) and 10% palladium on carbon (25 g) were added. Hydrogen (g) was charged into the reaction mixture. The reaction mixture was stirred under a hydrogen atmosphere for 3 h at room temperature. The reaction was vented to nitrogen and the solids were filtered out over celite. The filtered solution was concentrated under vacuum. The residue was subjected to purification by FCC eluting with ethyl acetate/petroleum ether (1:5). This afforded the title compound (28.5 g, 80%) as a yellow oil. MS: (ES, *m*/*z*): 174 [M+H]⁺.

### Step 3. (2S)-5-methoxy-2-methyl-1,2,3,4-tetrahydroquinoline

Into a 30-mL pressure tank reactor (50 atm), 5-methoxy-2-methylquinoline (4.0 g, 23.09 mmol) was dissolved in methanol (10 mL). Then Ru(OTf)(*η*6-hexamethylbenzene)((*S,S*)-TsDPEN) ([*N*-[(1*S*,2*S*)-2-(amino-κ*N*)-1,2-diphenylethyl]-4-methylbenzenesulfonamidato-κ*N*][(1,2,3,4,5,6-η)-1,2,3,4,5,6-hexamethylbenzene](1,1,1-trifluoromethanesulfonato-κ*O*)-ruthenium, prepared according to the procedure in J. Am. Chem. Soc. 2011, 133, 9878-9891) (150 mg, 0.23 mmol) was added. To the above hydrogen was introduced in. The resulting solution was stirred for 6 h at room temperature. The resulting mixture was concentrated under vacuum. The residue was subjected to purification by FCC eluting with ethyl acetate/petroleum ether (1:4). This afforded the title compound (3.0 g, 73%) as a yellow oil. MS: (ES, *m*/*z*): 178 [M+H]⁺.

### Step 4. methyl (S)-5-methoxy-2-methyl-3,4-dihydroquinoline-1(2H)-carboxylate

Into a 250-mL round-bottom flask, (2S)-5-methoxy-2-methyl-1,2,3,4-tetrahydroquinoline (18 g, 99.52 mmol) was dissolved in dichloromethane (100 mL). Then pyridine (23.6 g, 298.36 mmol) was added, followed by methyl carbonochloridate (9.4 g, 99.47 mmol). The resulting solution was stirred for 1 h at room temperature. The resulting solution was diluted with 100 mL of dichloromethane and washed with 3x200 mL of water. The organic layers were combined, dried over anhydrous sodium sulfate, filtered and concentrated under vacuum. The residue was subjected to purification by FCC eluting with ethyl acetate/petroleum ether (1:3). This afforded the title compound (21 g, 89%) as a yellow oil. MS: (ES, *m*/*z*): 236 [M+H]⁺.

### Step 5. methyl (S)-5-hydroxy-2-methyl-3,4-dihydroquinoline-1(2H)-carboxylate

Into a 500-mL 3-necked round-bottom flask, methyl (2S)-5-methoxy-2-methyl-1,2,3,4-tetrahydroquinoline-1-carboxylate (21 g, 89.36 mmol) was dissolved in dichloromethane (150 mL). Then boron tribromide (150 mL, 0.15 mol, 1 M in CH₂Cl₂) was added. The resulting solution was stirred for 1 h at room temperature. The reaction was then quenched by the addition of 300 mL of water. The resulting mixture was extracted with 3x300 mL of dichloromethane. The organic layers were combined, dried over anhydrous sodium sulfate, filtered and concentrated under vacuum. The residue was subjected to purification by FCC eluting with ethyl acetate/petroleum ether (1:2). This afforded the title compound (13.5 g, 68%) as a yellow solid. MS: (ES, *m*/*z*): 222 [M+H]⁺.

### Step 6. methyl (S)-2-methyl-5-(((trifluoromethyl)sulfonyl)oxy)-3,4-dihydroquinoline-1(2H)-carboxylate

Into a 250-mL round-bottom flask, methyl (2S)-5-hydroxy-2-methyl-1,2,3,4-tetrahydroquinoline-1-carboxylate (5 g, 18.08 mmol) was dissolved in dichloromethane (50 mL). Then pyridine (14.3 g, 180.78 mmol) and trifluoromethanesulfonic anhydride (10.2 g, 36.15 mmol) were added. The resulting solution was stirred for 1 h at room temperature. The resulting mixture was washed with 3x100 mL of water. The organic layers were combined, dried over anhydrous sodium sulfate, filtered and concentrated under vacuum. The residue was subjected to purification by FCC eluting with ethyl acetate/petroleum ether (1:3). This afforded the title compound (5.5 g, 86%) as a yellow oil. MS: (ES, *m*/*z*): 354 [M+H]⁺.

### Step 7. methyl (S)-5-((diphenylmethylene)amino)-2-methyl-3,4-dihydroquinoline-1(2H)-carboxylate

Into a 500-mL round-bottom flask purged and maintained with an inert atmosphere of nitrogen, methyl (2S)-2-methyl-5-[(trifluoromethane)sulfonyloxy]-1,2,3,4-tetrahydroquinoline - 1-carboxylate (23.5 g, 65.18 mmol) was dissolved in toluene (100 mL). Then diphenylmethanimine (17.9 g, 97.78 mmol), tris(dibenzylideneacetone)dipalladium-chloroform adduct (1.19 g, 1.30 mmol), (+/-)-2,2'-Bis(diphenylphosphino)-I,I'-binaphthyl (2.43 g, 3.90 mmol) and cesium carbonate (42.4 g, 130.13 mmol) were added. The resulting solution was stirred overnight at 100°C under nitrogen atmosphere. The reaction mixture was cooled and the solids were filtered out. The residue was subjected to purification by FCC eluting with ethyl acetate/petroleum ether (1:3). This afforded the title compound (33 g, 80%) as a yellow oil. MS: (ES, *m*/*z*): 385 [M+H]⁺.

### Step 8. methyl (S)-5-amino-2-methyl-3,4-dihydroquinoline-1(2H)-carboxylate

Into a 500-mL round-bottom flask, methyl (2S)-5-[(diphenylmethylidene)amino]-2-methyl-1,2,3,4-tetrahydroquinoline-1-carboxylate (33 g, 85.93 mmol) was dissolved in methanol (200 mL). Then sodium acetate (17 g, 207.23 mmol) and hydroxylamine hydrochloride (12.3 g, 177.00 mmol) were added. The resulting solution was stirred for 2 h at room temperature. The solids were filtered out. The resulting mixture was concentrated under vacuum. The residue was subjected to purification by FCC eluting with ethyl acetate/petroleum ether (1:2). This afforded the title compound (12.5 g, 66%) as a yellow solid. MS: (ES, *m*/*z*): 221 [M+H]⁺.

### Step 9. methyl (S)-5-amino-6-bromo-2-methyl-3,4-dihydroquinoline-1(2H)-carboxylate (Intermediate 1)

Into a 100-mL 3-necked round-bottom flask, methyl (2S)-5-amino-2-methyl-1,2,3,4-tetrahydroquinoline-1-carboxylate (1 g, 4.09 mmol) was dissolved in acetonitrile (20 mL). Then N-bromosuccinimide (730 mg, 4.10 mmol) was added. The resulting solution was stirred for 30 min at room temperature. The resulting mixture was concentrated under vacuum. The residue was subjected to purification by FCC eluting with ethyl acetate/petroleum ether (1:1). This afforded the title compound (1.1 g, 90%) as a yellow solid. MS: (ES, *m*/*z*): 299, 301 [M+H]⁺.
H-NMR: (400 MHz, CD30D, ppm): 7.19(d, J= 8.8 Hz, 1H), 6.84(d, J= 8.8 Hz, 1H), 4.73-4.69(m, 1H), 3.74(s, 3H), 2.64-2.57(m, 1H), 2.55-2.44(m, 1H), 2.12-2.05(m, 1H), 1.82-1.79(m, 1H), 1.17(d, *J*=6.9 Hz, 3H).

The synthetic schemes are presented for the synthesis of certain compounds herein disclosed. The process and results for the assays testing BET family bromodomain inhibition are also described.

### Example 1: methyl (S)-2-(2-(1H-pyrazol-1-yl)ethyl)-7-methyl-3-(2-(((1-methyl-1H-pyrazol-3-yl)methyl)amino)ethyl)-3,7,8,9-tetrahydro-6H-imidazo[4,5-f]quinoline-6-carboxylate

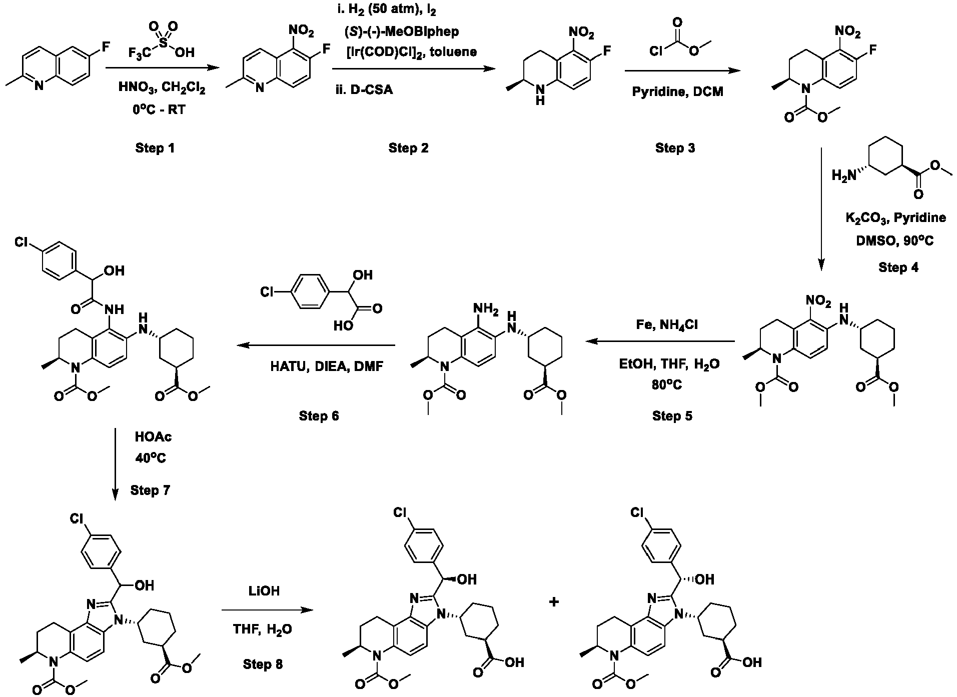

### Step 1. 6-fluoro-2-methyl-5-nitroquinoline

A solution of trifluoromethanesulfonic acid (82.0 mL, 0.923 mol) in HNO₃ (19.6 mL, 0.437 mol) was stirred for 20 min at 0 °C. This was followed by the addition of 6-fluoro-2-methylquinoline (50.0 g, 0.310 mol) in dichloromethane (300 mL) at 0 °C. The resulting mixture was stirred for 15 hours at room temperature (25 °C). The reaction mixture was diluted with water (300 mL). The pH value of the solution was adjusted to 8 with sodium bicarbonate (saturated aqueous solution). The resulting solution was extracted with dichloromethane (3 x 300 mL). The combined organic layers were dried over anhydrous sodium sulfate, filtered, and concentrated under vacuum. The residue was purified by silica gel chromatography (eluting with 1:4 ethyl acetate/petroleum ether) to afford 6-fluoro-2-methyl-5-nitroquinoline as a light yellow solid (60.0 g, 94%). LCMS (ES, *m*/*z*): 207 [M+H]⁺.

### Step 2. (2S)-6-fluoro-2-methyl-5-nitro-1,2,3,4-tetrahydroquinoline

A solution of (S)-(-)-MeO-BIPHEP (1.03 g, 1.77 mmol), chloro(1,5-cyclooctadiene)iridium(I) dimer (538 mg, 0.80 mmol) in toluene (100 mL) was stirred for 30 min at room temperature (25 °C) under an atmosphere of nitrogen. This was followed by the addition of I₂ (410 mg, 1.62 mmol), and 6-fluoro-2-methyl-5-nitroquinoline (33.0 g, 0.160 mol) in toluene (100 mL). The resulting mixture was stirred for 20 h at room temperature (25 °C) under hydrogen (50 atm). The resulting mixture was concentrated under vacuum and purified by silica gel chromatography (eluting with 1:1 ethyl acetate/petroleum ether) to afford the crude product (35.0 g). The crude product was dissolved in ethyl acetate (230 mL), followed by the addition of D-Camphorsulfonic acid (36.9 g, 0.158 mol). The resulting solution was stirred for 1 h at 60 °C and then cooled to room temperature. The solids were collected by filtration, and rinsed with ethyl acetate (120 mL). The solids were dissolved in water (50 mL). The pH value of the solution was adjusted to 8 with sodium bicarbonate (saturated aqueous solution). The resulting solution was extracted with ethyl acetate (3 x 120 mL). The combined organic layers was dried over anhydrous sodium sulfate, filtered, and concentrated under vacuum to afford (2S)-6-fluoro-2-methyl-5-nitro-1,2,3,4-tetrahydroquinoline as a red solid (25.5 g, 76%). LCMS (ES, *m*/*z*): 211 [M+H]⁺.

### Step 3. methyl (2S)-6-fluoro-2-methyl-5-nitro-1,2,3,4-tetrahydroquinoline-1-carboxylate

A solution of (2S)-6-fluoro-2-methyl-5-nitro-1,2,3,4-tetrahydroquinoline (25.3 g, 0.120 mol), pyridine (39.0 mL, 0.484 mol), and methyl carbonochloridate (18.7 mL, 0.242 mol) in dichloromethane (150 mL) was stirred for 3 h at room temperature (25 °C). The reaction was washed with IN hydrogen chloride (aq., 2 x 70 mL). The combined organic layers were dried over anhydrous sodium sulfate, filtered, and concentrated under vacuum to afford methyl (2S)-6-fluoro-2-methyl-5-nitro-1,2,3,4-tetrahydroquinoline-1-carboxylate as a yellow solid (29.8 g, 92%). LCMS (ES, *m*/*z*): 269 [M+H]⁺.

### Step 4. methyl (2S)-6-[[(1R,3R)-3-(methoxycarbonyl)cyclohexyl]amino]-2-methyl-5-nitro-1,2,3,4-tetrahydroquinoline-1-carboxylate

A solution of methyl (2S)-6-fluoro-2-methyl-5-nitro-1,2,3,4-tetrahydroquinoline-1-carboxylate (29.6 g, 0.110 mol), pyridine (29.6 mL, 0.368 mol), potassium carbonate (30.5 g, 0.220 mol), and methyl (1R,3R)-3-aminocyclohexane-1-carboxylate (25.6 g, 162.84 mmol) in DMSO (270 mL) was stirred for 15 h at 90 °C and then cooled to room temperature. The reaction was quenched by the addition of water (200 mL) and extracted with ethyl acetate (3 x 300 mL). The combined organic layers were dried over anhydrous sodium sulfate, filtered, and concentrated under vacuum. The resulting crude product was purified by silica gel chromatography (eluting with 1:1 ethyl acetate/petroleum ether) to afford methyl (2S)-6-[[(1R,3R)-3-(methoxycarbonyl)cyclohexyl]amino]-2-methyl-5-nitro-1,2,3,4-tetrahydroquinoline-1-carboxylate as a red oil (32 g, 72%). LCMS (ES, *m*/*z*): 406 [M+H]⁺.

### Step 5. methyl (2S)-5-amino-6-[[(1R,3R)-3-(methoxycarbonyl)cyclohexyl]amino]-2-methyl-1,2,3,4-tetrahydroquinoline-1-carboxylate

A solution of methyl (2S)-2-methyl-5-nitro-6-[[(1R,3R)-4-(methoxycarbonyl)cyclohexyl]amino]-1,2,3,4-tetrahydroquinoline-1-carboxylate (31.0 g, 76.46 mmol), NH₄Cl (24.3 g, 454.28 mmol), and Fe (powder, 64.3 g, 1.15 mol) in tetrahydrofuran (300 mL), ethanol (300 mL), water (100 mL) was stirred for 1 h at 80 °C and then cooled to room temperature. The solids were filtered out by filtration. The resulting solution was diluted with water (300 mL) and extracted with ethyl acetate (3 x 400 mL). The combined organic layers were dried over anhydrous sodium sulfate, filtered, and concentrated under vacuum to afford methyl (2S)-5-((R)-2-hydroxy-2-phenylacetamido)-6-[[(1R,3R)-3-(methoxycarbonyl)cyclohexyl] amino]-2-methyl-1,2,3,4-tetrahydroquinoline-1-carboxylate as a dark green solid (27.5 g, 92%). LCMS (ES, *m*/*z*): 376 [M+H]⁺.

### Step 6. methyl (2S)-5-[2-(4-chlorophenyl)-2-hydroxyacetamido]-6-[[(1R,3R)-3-(methoxycarbonyl)cyclohexyl]amino]-2-methyl-1,2,3,4-tetrahydroquinoline-1-carboxylate

A solution of 2-(4-chlorophenyl)-2-hydroxyacetic acid (112 mg, 0.60 mmol), HATU (304 mg, 0.80 mmol), methyl (2S)-5-amino-6-[[(1R,3R)-3-(methoxycarbonyl)cyclohexyl]amino]-2-methyl-1,2,3,4-tetrahydroquinoline-1-carboxylate (150 mg, 0.40 mmol), and DIEA (155 mg, 1.20 mmol) in N,N-dimethylformamide (2 mL) was stirred for 15 h at room temperature (25 °C). The resulting solution was diluted with water (30 mL), and extracted with ethyl acetate (3 x 50 mL). The organic layers were combined and washed with brine (2 x 25 mL). The combined organic layers were dried over anhydrous sodium sulfate, filtered, and concentrated under vacuum. The resulting crude product was purified by silica gel chromatography (eluting with 1:1 ethyl acetate/petroleum ether) to afford methyl (2S)-5-[2-(4-chlorophenyl)-2-hydroxyacetamido]-6-[[(1R,3R)-3-(methoxycarbonyl)cyclohexyl]amino]-2-methyl-1,2,3,4-tetrahydroquinoline-1-carboxylate as yellow oil (70.0 mg, 32%). LCMS (ES, m/z): 544 [M+H]⁺.

### Step 7. methyl (7S)-2-[(4-chlorophenyl)(hydroxy)methyl]-3-[(1R,3R)-3-(methoxycarbonyl)cyclohexyl]-7-methyl-3H,6H,7H,8H,9H-imidazo[4,5-f]quinoline-6-carboxylate

A solution of methyl (2S)-5-[2-(4-chlorophenyl)-2-hydroxyacetamido]-6-[[(1R,3R)-3-(methoxycarbonyl)cyclohexyl]amino]-2-methyl-1,2,3,4-tetrahydroquinoline-1-carboxylate (60.0 mg, 0.11 mmol) in AcOH (2 mL) was stirred for 15 h at 40 °C and then cooled to room temperature. The reaction mixture was diluted with water (10 mL). The pH value of the solution was adjusted to 8 with sodium bicarbonate (saturated aqueous solution). The resulting solution was extracted with ethyl acetate (3 x 15 mL). The organic layers were combined and dried over anhydrous sodium sulfate, filtered, and concentrated under vacuum. The resulting crude product was purified by silica gel chromatography (eluting with 1:1 ethyl acetate/petroleum ether) to afford methyl (7S)-2-[(4-chlorophenyl)(hydroxy)methyl]-3-[(1R,3R)-3-(methoxycarbonyl)cyclohexyl]-7-methyl-3H,6H,7H,8H,9H-imidazo[4,5-f]quinoline-6-carboxylate as yellow oil (46.0 mg, 79%). LCMS (ES, *m*/*z*): 526 [M+H]⁺.

### Step 8. (1R,3R)-3-[(7S)-2-[(R)-(4-chlorophenyl)(hydroxy)methyl]-6-(methoxycarbonyl)-7-methyl-3H,6H,7H,8H,9H-imidazo[4,5-f]quinolin-3-yl]cyclohexane-1-carboxylic acid; (1R,3R)-3-[(7S)-2-[(S)-(4-chlorophenyl)(hydroxy)methyl]-6-(methoxycarbonyl)-7-methyl-3H,6H,7H,8H,9H-imidazo[4,5-f]quinolin-3-yl]cyclohexane-1-carboxylic acid

A solution of methyl (7S)-2-[(4-chlorophenyl)(hydroxy)methyl]-3-[(1R,3R)-3-(methoxycarbonyl)cyclohexyl]-7-methyl-3H,6H,7H,8H,9H-imidazo[4,5-f]quinoline-6-carboxylate (50.0 mg, 0.10 mmol), and LiOH (11.4 mg, 0.48 mmol) in tetrahydrofuran (1 mL) and water (1 mL) was stirred for 15 h at 25 °C. The resulting mixture was concentrated under vacuum. The crude product was purified by Prep-HPLC (Column: XBridge Shield RP18 OBD Column, 5um, 19 x 150 mm; Mobile Phase, A: water (containing 10 mmol/L NH₄HCO₃) and B: ACN (10% to 37% over 12 min); Detector: UV 254 nm). The product fractions were lyophilized to afford (1R,3R)-3-[(7S)-2-[(R)-(4-chlorophenyl)(hydroxy)methyl]-6-(methoxycarbonyl)-7-methyl-3H,6H,7H,8H,9H-imidazo[4,5-f]quinolin-3-yl]cyclohexane-1-carboxylic acid (413) as a white solid (10.5 mg, 43%); and (1R,3R)-3-[(7S)-2-[(S)-(4-chlorophenyl)(hydroxy)methyl]-6-(methoxycarbonyl)-7-methyl-3H,6H,7H,8H,9H-imidazo[4,5-f]quinolin-3-yl]cyclohexane-1-carboxylic acid (501) as a white solid (7.0 mg, 29%).
**First eluting isomer (413):** ¹H-NMR (CD₃OD, 400 MHz) δ (ppm): 7.49 (d, *J* = 9.0 Hz, 1H), 7.42-7.33 (m, 5H), 6.19 (s, 1H), 4.92-4.90 (m, 1H), 4.82-4.72 (m, 1H), 3.79 (s, 3H), 3.34-3.20 (m, 1H), 3.02-2.94 (m, 1H), 2.90-2.87 (m, 1H), 2.36-2.09 (m, 4H), 1.99-1.96 (m, 1H), 1.80-1.42 (m, 5H), 1.16 (d, *J* = 6.6 Hz, 3H). LCMS (ES, *mlz*): 512 [M+H]⁺.
**Second eluting isomer (501):** ¹H-NMR (CD₃OD, 400 MHz) δ (ppm): 7.52-7.33 (m, 6H), 6.22 (s, 1H), 4.84-4.73 (m, 2H), 3.78 (s, 3H), 3.27-3.16 (m, 1H), 3.04-2.92 (m, 1H), 2.90-2.88 (m, 1H), 2.46-2.35 (m, 2H), 2.30-2.22 (m, 1H), 2.15-2.02 (m, 2H), 1.82-1.71 (m, 1H), 1.63-1.55 (m, 2H), 1.40-1.28 (m, 1H), 1.15 (d, *J* = 6.6 Hz, 4H). LCMS (ES, *m*/*z*): 512 [M+H]⁺.

The compounds listed in Figure 1 were prepared using standard chemical manipulations and procedures similar to those described herein. In Figure 1, "Eluted Isomer" refers to the order in which the compound eluted by preparative HPLC.

### Example 2: Compounds 424 and 660: (1R,3R)-3-[(7S)-2-[(R)-(5-fluoro-2-methoxyphenyl)(hydroxy)methyl]-6-(methoxycarbonyl)-7-methyl-3H,6H,7H,8H,9H-imidazo[4,5-f]quinolin-3-yl]cyclohexane-1-carboxylic acid (424); (1R,3R)-3-[(7S)-2-[(S)-(5-fluoro-2-methoxyphenyl)(hydroxy)methyl]-6-(methoxycarbonyl)-7-methyl-3H,6H,7H,8H,9H-imidazo[4,5-f]quinolin-3-yl]cyclohexane-1-carboxylic acid (660)

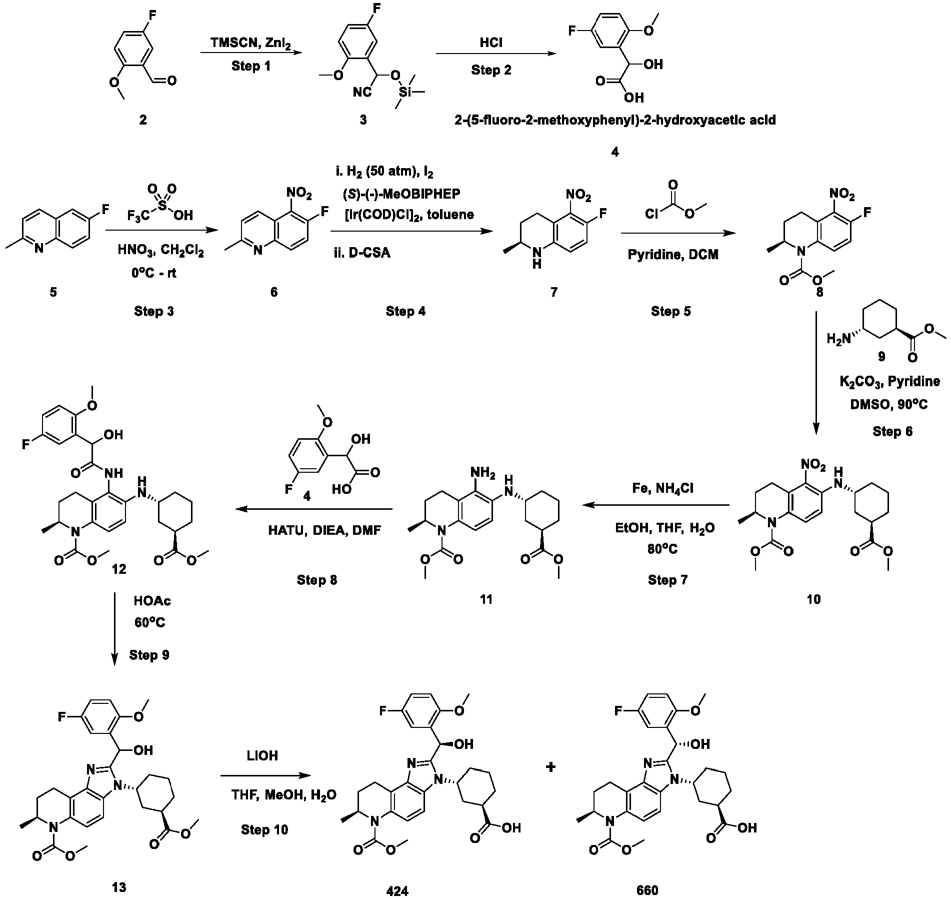

### Step 1. 2-(5-fluoro-2-methoxyphenyl)-2-[(trimethylsilyl)oxylacetonitrile

A solution of ZnI₂ (1.6 mg, 0.01 mmol), 5-fluoro-2-methoxybenzaldehyde (1.54 g, 9.99 mmol) in trimethylsilanecarbonitrile (1.5 mL, 11.25 mmol) was stirred for 1 h at room temperature. The resulting mixture was concentrated under vacuum. The resulting crude product was purified by silica gel chromatography (eluting with 1:1 ethyl acetate/petroleum ether) to afford 2-(5-fluoro-2-methoxyphenyl)-2-[(trimethylsilyl)oxy]acetonitrile as a white solid (2.0 g, 79%).

### Step 2. 2-(5-fluoro-2-methoxyphenyl)-2-hydroxyacetic acid

A solution of 2-(5-fluoro-2-methoxyphenyl)-2-[(trimethylsilyl)oxy]acetonitrile (1.50 g, 5.92 mmol) in hydrochloric acid (10 mL, 12M) was stirred for 1 h at 25 °C, and then stirred for 2 h at 70 °C. The reaction mixture was cooled and concentrated under vacuum. The crude product was purified by reverse phase chromatography (Column: C18; Mobile phase, A: water (containing 0.05% TFA) and B: ACN (5% to 20% over 30 min); Detector, UV 254 nm) to afford 2-(5-fluoro-2-methoxyphenyl)-2-hydroxyacetic acid as a white solid (1.10 g, 93%).

### Step 3. 6-fluoro-2-methyl-5-nitroquinoline

A solution of trifluoromethanesulfonic acid (82.0 mL, 0.923 mol) in HNO₃ (19.6 mL, 0.437 mol) was stirred for 20 min at 0 °C. This was followed by the addition of 6-fluoro-2-methylquinoline (50.0 g, 0.310 mol) in dichloromethane (300 mL) at 0 °C. The resulting mixture was stirred for 15 h at room temperature (25 °C). The reaction mixture was diluted with water (300 mL). The pH value of the solution was adjusted to 8 with sodium bicarbonate (saturated aqueous solution). The resulting solution was extracted with dichloromethane (3 x 300 mL). The combined organic layers were dried over anhydrous sodium sulfate, filtered, and concentrated under vacuum. The residue was purified by silica gel chromatography (eluting with 1:4 ethyl acetate/petroleum ether) to afford 6-fluoro-2-methyl-5-nitroquinoline as a light yellow solid (60.0 g, 94%). LCMS (ES, *m*/*z*): 207 [M+H]⁺.

### Step 4. (2S)-6-fluoro-2-methyl-5-nitro-1,2,3,4-tetrahydroquinoline

A solution of (S)-(-)-MeO-BIPHEP (1.03 g, 1.77 mmol), chloro(1,5-cyclooctadiene)iridium(I) dimer (538 mg, 0.80 mmol) in toluene (100 mL) was stirred for 30 min at room temperature (25 °C) under an atmosphere of nitrogen. This was followed by the addition of I₂ (410 mg, 1.62 mmol), 6-fluoro-2-methyl-5-nitroquinoline (33.0 g, 0.160 mol) in toluene (100 mL). The resulting mixture was stirred for 20 h at room temperature (25 °C) under hydrogen (50 atm). The resulting mixture was concentrated under vacuum and purified by silica gel chromatography (eluting with 1:1 ethyl acetate/petroleum ether) to afford the crude product (35.0 g). The crude product was dissolved in ethyl acetate (230 mL), followed by the addition of D-Camphorsulfonic acid (36.9 g, 0.158 mol). The resulting solution was stirred for 1 h at 60 °C and then cooled to room temperature. The solids were collected by filtration, and rinsed with ethyl acetate (120 mL). The solids were dissolved in water (50 mL). The pH value of the solution was adjusted to 8 with sodium bicarbonate (saturated aqueous solution). The resulting solution was extracted with ethyl acetate (3 x 120 mL). The combined organic layers were dried over anhydrous sodium sulfate, filtered, and concentrated under vacuum to afford (2S)-6-fluoro-2-methyl-5-nitro-1,2,3,4-tetrahydroquinoline as a red solid (25.5 g, 76%). LCMS (ES, *m*/*z*): 211 [M+H]⁺.

### Step 5. methyl (2S)-6-fluoro-2-methyl-5-nitro-1,2,3,4-tetrahydroquinoline-1-carboxylate

A solution of (2S)-6-fluoro-2-methyl-5-nitro-1,2,3,4-tetrahydroquinoline (25.3 g, 0.120 mol), pyridine (39.0 mL, 0.484 mol), methyl carbonochloridate (18.7 mL, 0.242 mol) in dichloromethane (150 mL) was stirred for 3 h at room temperature (25 °C). The reaction was washed with 1M hydrochloric acid (2 x 70 mL). The combined organic layers were dried over anhydrous sodium sulfate, filtered, and concentrated under vacuum to afford methyl (2S)-6-fluoro-2-methyl-5-nitro-1,2,3,4-tetrahydroquinoline-1-carboxylate as a yellow solid (29.8 g, 92%). LCMS (ES, *m*/*z*): 269 [M+H]⁺.

### Step 6. methyl (2S)-6-[[(1R,3R)-3-(methoxycarbonyl)cyclohexyl]amino]-2-methyl-5-nitro-1,2,3,4-tetrahydroquinoline-1-carboxylate

A solution of methyl (2S)-6-fluoro-2-methyl-5-nitro-1,2,3,4-tetrahydroquinoline-1-carboxylate (29.6 g, 0.110 mol), pyridine (29.6 mL, 0.368 mol), potassium carbonate (30.5 g, 0.220 mol), methyl (1R,3R)-3-aminocyclohexane-1-carboxylate (25.6 g, 162.84 mmol) in DMSO (270 mL) was stirred for 15 h at 90 °C and then cooled to room temperature. The reaction was quenched by the addition of water (200 mL) and extracted with ethyl acetate (3 x 300 mL). The combined organic layers were dried over anhydrous sodium sulfate, filtered, and concentrated under vacuum. The resulting crude product was purified by silica gel chromatography (eluting with 1:1 ethyl acetate/petroleum ether) to afford methyl (2S)-6-[[(1R,3R)-3-(methoxycarbonyl) cyclohexyl]amino]-2-methyl-5-nitro-1,2,3,4-tetrahydroquinoline-1-carboxylate as a red oil (32 g, 72%). LCMS (ES, *m*/*z*): 406 [M+H]⁺.

### Step 7. methyl (2S)-5-amino-6-[[(1R,3R)-3-(methoxycarbonyl)cyclohexyl]amino]-2-methyl-1,2,3,4-tetrahydroquinoline-1-carboxylate

A solution of methyl (2S)-6-[[(lR,3R)-3-(methoxycarbonyl)cyclohexyl]amino]-2-methyl-5-nitro-1,2,3,4-tetrahydroquinoline-1-carboxylate (31.0 g, 76.46 mmol), NH₄Cl (24.3 g, 454.28 mmol), Fe (64.3 g, 1.15 mol) in tetrahydrofuran (300 mL), ethanol (300 mL), and water (100 mL) was stirred for 1 h at 80 °C and then cooled to room temperature. The solids were filtered out by filtration. The resulting solution was diluted with water (300 mL) and extracted with ethyl acetate (3 x 400 mL). The combined organic layers were dried over anhydrous sodium sulfate, filtered, and concentrated under vacuum to afford methyl (2S)-5-amino-6-[[(1R,3R)-3-(methoxycarbonyl)cyclohexyl]amino]-2-methyl-1,2,3,4-tetrahydroquinoline-1-carboxylate as a dark green solid (27.5 g, 92%). LCMS (ES, *m*/*z*): 376 [M+H]⁺.

### Step 8. methyl (2S)-5-[2-(5-fluoro-2-methoxyphenyl)-2-hydroxyacetamido]-6-[[(1R,3R)-3-(methoxycarbonyl)cyclohexyl]amino]-2-methyl-1,2,3,4-tetrahydroquinoline-1-carboxylate

A solution of 2-(5-fluoro-2-methoxyphenyl)-2-hydroxyacetic acid (240 mg, 1.20 mmol), HATU (228 mg, 0.60 mmol), methyl (2S)-5-amino-6-[[(1R,3R)-3-(methoxycarbonyl) cyclohexyl]amino]-2-methyl-1,2,3,4-tetrahydroquinoline-1-carboxylate (150 mg, 0.40 mmol), DIEA (0.19 mL, 1.20 mmol) in N,N-dimethylformamide (10 mL) was stirred for 1 h at 25 °C. The resulting solution was diluted with H₂O (10 mL). The resulting solution was extracted with ethyl acetate (3x15 mL) and the organic layers combined. The resulting mixture was washed with brine (2x20 mL). The mixture was dried over anhydrous sodium sulfate and concentrated under vacuum. The resulting crude product was purified by silica gel chromatography (eluting with 3:2 ethyl acetate/petroleum ether) to afford methyl (2S)-5-[2-(5-fluoro-2-methoxyphenyl)-2-hydroxyacetamido]-6-[[(1R,3R)-3-(methoxycarbonyl)cyclohexyl]amino]-2-methyl-1,2,3,4-tetrahydroquinoline-1-carboxylate as a yellow solid (180 mg, 81%). LCMS (ES, *m*/*z*): 558 [M+H]⁺.

### Step 9. methyl (7S)-2-[(5-fluoro-2-methoxyphenyl)(hydroxy)methyl]-3-[(1R,3R)-3-(methoxycarbonyl)cyclohexyl]-7-methyl-3H,6H,7H,8H,9H-imidazo[4,5-f]quinoline-6-carboxylate.

A solution of methyl (2S)-5-[2-(5-fluoro-2-methoxyphenyl)-2-hydroxyacetamido]-6-[[(1R,3R)-3-(methoxycarbonyl)cyclohexyl]amino]-2-methyl-1,2,3,4-tetrahydroquinoline-1-carboxylate (180 mg, 0.32 mmol) in AcOH (8 mL) was stirred for overnight at 60 °C. The reaction mixture was cooled and concentrated under vacuum. The resulting crude product was purified by silica gel chromatography (eluting with 1:1 ethyl acetate/petroleum ether) to afford methyl (7S)-2-[(5-fluoro-2-methoxyphenyl)(hydroxy)methyl]-3-[(1R,3R)-3-(methoxycarbonyl)cyclohexyl]-7-methyl-3H,6H,7H,8H,9H-imidazo[4,5-f]quinoline-6-carboxylate as a yellow solid (120 mg, 69%). LCMS (ES, *m*/*z*): 540 [M+H]⁺.

### Step 10. (1R,3R)-3-[(7S)-2-[(R)-(5-fluoro-2-methoxyphenyl)(hydroxy)methyl]-6-(methoxycarbonyl)-7-methyl-3H,6H,7H,8H,9H-imidazo[4,5-f]quinolin-3-yl]cyclohexane-1-carboxylic acid; (1R,3R)-3-[(7S)-2-[(S)-(5-fluoro-2-methoxyphenyl)(hydroxy)methyl]-6-(methoxycarbonyl)-7-methyl-3H,6H,7H,8H,9H-imidazo[4,5-f]quinolin-3-yl]cyclohexane-1-carboxylic acid

A solution of methyl (7S)-2-[(5-fluoro-2-methoxyphenyl)(hydroxy)methyl]-3-[(1R,3R)-3-(methoxycarbonyl)cyclohexyl]-7-methyl-3H,6H,7H,8H,9H-imidazo[4,5-f]quinoline-6-carboxylate (120 mg, 0.22 mmol), and LiOH (16 mg, 0.67 mmol) in tetrahydrofuran (2.0 mL), methanol (2.0 mL) and water (2.0 mL) was stirred overnight at 25 °C. The resulting mixture was concentrated under vacuum. The crude product was purified by Prep-HPLC (Column, XBridge Prep C18 OBD Column, 19x150 mm, 5um; Mobile phase, A: water (containing 10 mmol/L NH₄HCO₃) and B: ACN (15.0% to 29.0% over 14 min); Detector, UV 220/254nm). The product was separated by Chiral-Prep-HPLC (Column, CHIRALPAK IE, 2x25cm, 5 um; Mobile phase, A: Hex (containing 0.1%FA) and B: ethanol (hold 50.0% ethanol over 12 min); Detector, UV 220/254 nm). The product fractions were concentrated to afford (1R,3R)-3-[(7S)-2-[(R)-(5-fluoro-2-methoxyphenyl)(hydroxy)methyl]-6-(methoxycarbonyl)-7-methyl-3H,6H,7H,8H,9H-imidazo [4,5-f]quinolin-3-yl]cyclohexane-1-carboxylic acid as a white solid (23.6 mg, 20%); and (1R,3R)-3-[(7S)-2-[(S)-(5-fluoro-2-methoxyphenyl)(hydroxy)methyl]-6-(methoxycarbonyl)-7-methyl-3H,6H,7H,8H,9H-imidazo[4,5-f]quinolin-3-yl]cyclohexane-1-carboxylic acid as a white solid (23.8 mg, 20%). Stereoisomeric purity was determined via HPLC: Column: CHIRALPAK IE-3, Column size: 0.46 x 5 cm; 3 µm; Mobile phase: Hex (0.1%FA) : EtOH = 50 : 50, Flow: 1.0 ml/min.
**First eluting isomer (424):** 1H-NMR (CD30D, 400 MHz) δ (ppm): 7.56-7.47 (m, 1H), 7.47-7.31 (m, 1H), 7.21-7.09 (m, 1H), 7.09-6.89 (m, 2H), 6.53(s, 1H), 4.81-4.61(m, 2H), 3.85(s, 3H), 3.78(s, 3H), 3.31-3.18(m, 1H), 3.06-2.82 (m, 2H), 2.57-2.41 (m, 1H), 2.41-2.31 (m, 1H), 2.31-2.09 (m, 3H), 1.83-1.58 (m, 3H), 1.49-1.21 (m, 2H), 1.16 (d, J = 6.8 Hz, 3H). LCMS (ES, m/z): 526 [M+H]+.
**Second eluting isomer (660):** 1H-NMR (CD30D, 400 MHz) δ (ppm): 7.69-7.44 (m, 2H), 7.44-7.29 (m, 1H), 7.12-6.99 (m, 1H), 6.98-6.82 (m, 1H), 6.37(s, 1H), 5.03-4.91(m, 1H), 4.81-4.69(m, 1H), 3.78(s, 3H), 3.61(s, 3H), 3.22-3.04(m, 1H), 3.02-2.87 (m, 2H), 2.54-2.41 (m, 1H), 2.41-2.27 (m, 1H), 2.27-2.08 (m, 3H), 1.82-1.58 (m, 3H), 1.58-1.41 (m, 2H), 1.14 (d, J = 6.4 Hz, 3H). LCMS (ES, m/z): 526 [M+H]+.

In a preferred embodiment the disclosure provides the first eluting isomer obtained from Step 10 of the process described in Example 2 above, or a pharmaceutically acceptable salt thereof. In a preferred embodiment the disclosure provides compound 424 having the following structure: 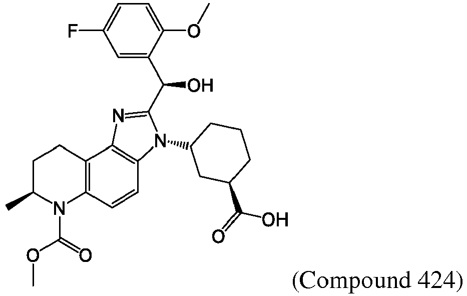 or a pharmaceutically acceptable salt, hydrate, solvate, or tautomer thereof.

In some embodiments, the disclosure provides a pharmaceutical composition comprising compound 424 of the foregoing structure or a pharmaceutically acceptable salt thereof, at a purity of at least 90%, for example greater than 90%, 91%, 92%, 93%, 94%, 95%, 96%, 97%, 98%, or 99% relative to one or more of its related stereoisomers. For example, the disclosure provides the compound 424 of the foregoing structure or a pharmaceutically acceptable salt thereof, at a purity of at least 90%, e.g. greater than 90%, 91%, 92%, 93%, 94%, 95%, 96%, 97%, 98%, or 99% purity relative to compound 660 and optionally other stereoisomers of compound 424 depicted below. In some embodiments, the disclosure provides a pharmaceutical composition comprising compound 424 of the foregoing structure or a pharmaceutically acceptable salt thereof, at a purity of at least 95%.

A composition of Formula (I) can comprise a compound of one or more of Formula (I-a), (I-b), (I-c), (I-d), (I-e), (I-f), (I-g), (I-h), (I-i), (I-j), (I-k), (I-1), (I-m), (I-n), and/or (I-o). For example, in some embodiments the disclosure provides a composition comprising compound 424 of the foregoing structure or a pharmaceutically acceptable salt thereof at a purity of at least 90% wherein the composition comprises less than 10%, e.g. less than 9%, less than 8%, less than 7%, less than 6%, less than 5%, less than 4%, less than 3%, less than 2% or less than 1%, collectively of one or more of the following stereoisomers of compound 424, represented as Formulae (II-a) - (II-o) below:

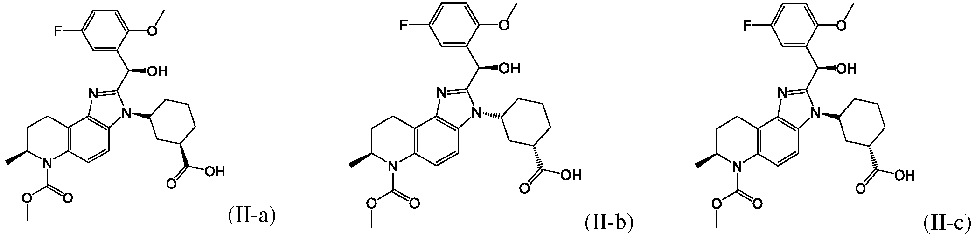

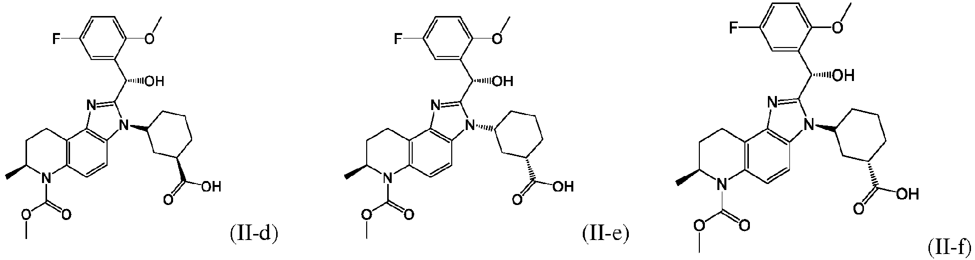

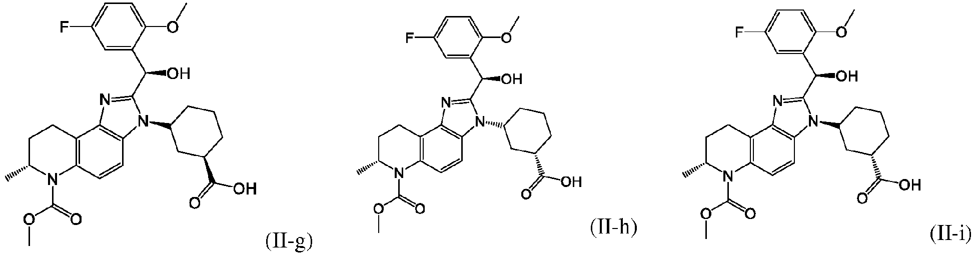

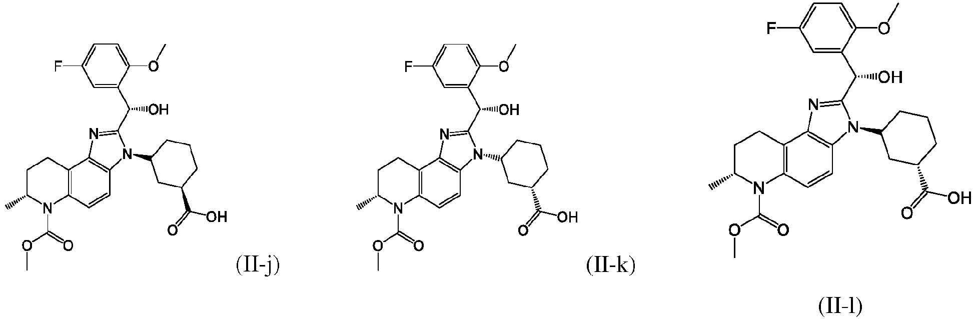

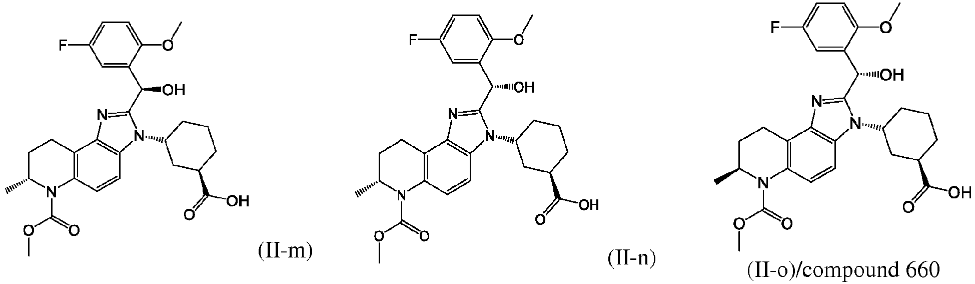

In any of the foregoing embodiments, the percentage purity recited may be determined by HPLC. In some embodiments the percentage purity is determined using the following HPLC method:
**Sample Preparation:**
   Prepare 0.2mg/mL in 70/30 Water/Acetonitrile.
**LCMS Information:**
   **Instruments:**
      MS: Waters QDa MS
      HPLC: Waters Alliance e2595
      UV: Waters 2998 PDA
**Conditions:**
   Mobile Phase A: 10mM Ammonium acetate
   Mobile Phase B: Acetonitrile
   Column: Waters XSelect Phenyl-Hexyl, 3.5 µm, 4.6x150 mm
   Column Temperature: 35°C
   LC Gradient:
   Runtime: 25 min
   LC Flow Rate: 1 mL/min
   UV Wavelength: 238 nm
   Ionization Mode: Electrospray Ionization+ive
   Injection Volume: 8µL

For instance, the disclosure provides a pharmaceutical composition comprising compound 424 or a pharmaceutically acceptable salt thereof at a purity of at least 95% as determined by the above HPLC method. The disclosure also provides a pharmaceutical composition comprising compound 424 at a purity of at least 95% as determined by the above HPLC method.

The disclosure provides a compound of Formula II obtained by the foregoing method exemplified in Example 2: 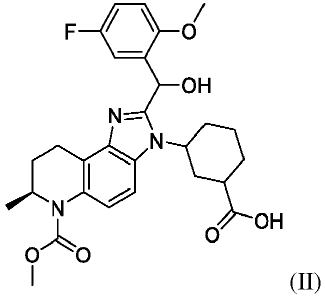 or a pharmaceutically acceptable salt, enantiomer, hydrate, solvate, isomer or tautomer thereof.

It will be apparent to the skilled reader that each of the stereoisomers of the compound of Formula (II) can be obtained by varying the stereochemistry of the appropriate reagents utilized in the method of Example 2 above. For instance, by adjusting the reagent used in Step 4 of Example 2, compounds such as those of Formulae (II-m) and (II-n) can be synthesized. Similarly, in Step 6 of Example 2, the regent methyl (1S,3R)-3-aminocyclohexane-1-carboxylate can be used in place of methyl (1R,3R)-3-aminocyclohexane-1-carboxylate to obtain compounds of Formulae (II-b) and (II-e). It will be apparent to the skilled reader that by making a combination of these types of modifications to the process set out in Example 2, each of compounds (II-a) to (II-o) depicted above can be synthesized.

### Example 3: (1R,3R)-3-[(7S)-2-[(R)-hydroxy(phenyl)methyl]-6-(methoxycarbonyl)-7-methyl-3H,6H,7H,8H,9H-imidazo[4,5-f]quinolin-3-yl]cyclohexane-1-carboxylic acid (462)

Compositions comprising Compound **462** can be prepared as shown in the scheme below:

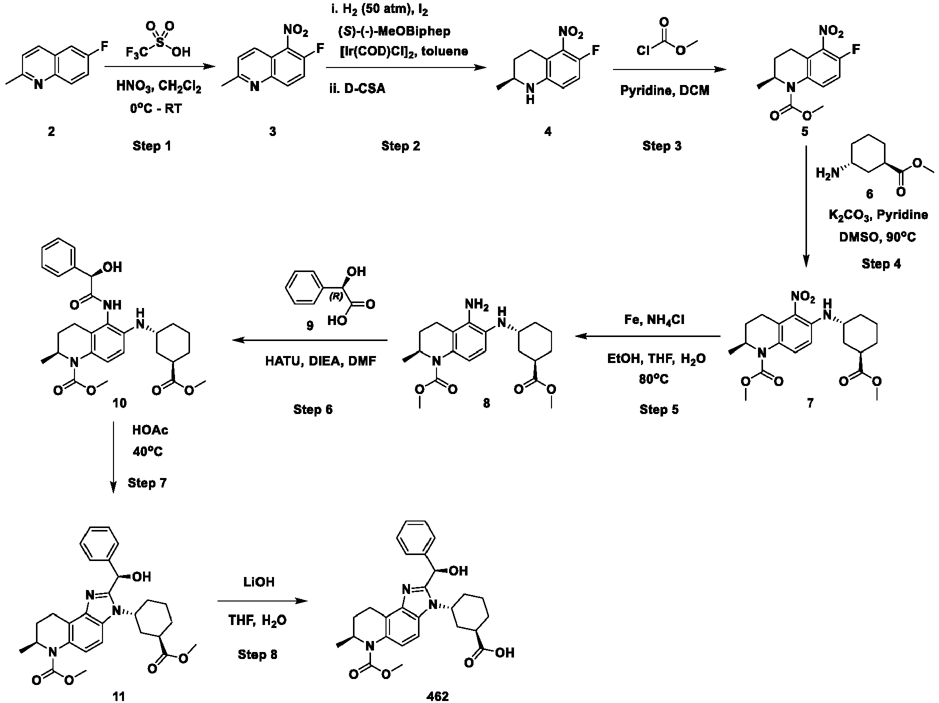

### Step 1. 6-fluoro-2-methyl-5-nitroquinoline

A solution of trifluoromethanesulfonic acid (82.0 mL, 0.923 mol) in HNO₃ (19.6 mL, 0.437 mol) was stirred for 20 min at 0 °C. This was followed by the addition of 6-fluoro-2-methylquinoline (50.0 g, 0.310 mol) in dichloromethane (300 mL) at 0 °C. The resulting mixture was stirred for 15 h at room temperature (25 °C). The reaction mixture was diluted with water (300 mL). The pH value of the solution was adjusted to 8 with sodium bicarbonate (saturated aqueous solution). The resulting solution was extracted with dichloromethane (3 x 300 mL). The combined organic layers were dried over anhydrous sodium sulfate, filtered, and concentrated under vacuum. The residue was purified by silica gel chromatography (eluting with 1:4 ethyl acetate/petroleum ether) to afford 6-fluoro-2-methyl-5-nitroquinoline as a light yellow solid (60.0 g, 94%). LCMS (ES, *m*/*z*): 207 [M+H]⁺.

### Step 2. (2S)-6-fluoro-2-methyl-5-nitro-1,2,3,4-tetrahydroquinoline

A solution of (S)-(-)-MeO-BIPHEP (1.03 g, 1.77 mmol), chloro(1,5-cyclooctadiene)iridium(I) dimer (538 mg, 0.80 mmol) in toluene (100 mL) was stirred for 30 min at room temperature (25 °C) under an atmosphere of nitrogen. This was followed by the addition of I₂ (410 mg, 1.62 mmol), 6-fluoro-2-methyl-5-nitroquinoline (33.0 g, 0.160 mol) in toluene (100 mL). The resulting mixture was stirred for 20 h at room temperature (25 °C) under hydrogen (50 atm). The resulting mixture was concentrated under vacuum and purified by silica gel chromatography (eluting with 1:1 ethyl acetate/petroleum ether) to afford the crude product (35.0 g). The crude product was dissolved in ethyl acetate (230 mL), followed by the addition of D-Camphorsulfonic acid (36.9 g, 0.158 mol). The resulting solution was stirred for 1 h at 60 °C and then cooled to room temperature. The solids were collected by filtration, and rinsed with ethyl acetate (120 mL). The solids were dissolved in water (50 mL). The pH value of the solution was adjusted to 8 with sodium bicarbonate (saturated aqueous solution). The resulting solution was extracted with ethyl acetate (3 x 120 mL). The combined organic layers were dried over anhydrous sodium sulfate, filtered, and concentrated under vacuum to afford (2S)-6-fluoro-2-methyl-5-nitro-1,2,3,4-tetrahydroquinoline as a red solid (25.5 g, 76%). LCMS (ES, *m*/*z*): 211 [M+H]⁺.

### Step 3. methyl (2S)-6-fluoro-2-methyl-5-nitro-1,2,3,4-tetrahydroquinoline-1-carboxylate

A solution of (2S)-6-fluoro-2-methyl-5-nitro-1,2,3,4-tetrahydroquinoline (25.3 g, 0.120 mol), pyridine (39.0 mL, 0.484 mol), methyl carbonochloridate (18.7 mL, 0.242 mol) in dichloromethane (150 mL) was stirred for 3 h at room temperature (25 °C). The reaction was washed with 1M hydrogen chloride (2 x 70 mL). The combined organic layers were dried over anhydrous sodium sulfate, filtered, and concentrated under vacuum to afford methyl (2S)-6-fluoro-2-methyl-5-nitro-1,2,3,4-tetrahydroquinoline-1-carboxylate as a yellow solid (29.8 g, 92%). LCMS (ES, *m*/*z*): 269 [M+H]⁺.

### Step 4. methyl (2S)-6-[[(1R,3R)-3-(methoxycarbonyl)cyclohexyl]amino]-2-methyl-5-nitro-1,2,3,4-tetrahydroquinoline-1-carboxylate

A solution of methyl (2S)-6-fluoro-2-methyl-5-nitro-1,2,3,4-tetrahydroquinoline-1-carboxylate (29.6 g, 0.110 mol), pyridine (29.6 mL, 0.368 mol), potassium carbonate (30.5 g, 0.220 mol), methyl (1R,3R)-3-aminocyclohexane-1-carboxylate (25.6 g, 162.84 mmol) in DMSO (270 mL) was stirred for 15 h at 90 °C and then cooled to room temperature. The reaction was quenched by the addition of water (200 mL) and extracted with ethyl acetate (3 x 300 mL). The combined organic layers were dried over anhydrous sodium sulfate, filtered, and concentrated under vacuum. The resulting crude product was purified by silica gel chromatography (eluting with 1:1 ethyl acetate/petroleum ether) to afford methyl (2S)-6-[[(1R,3R)-3-(methoxycarbonyl)cyclohexyl]amino]-2-methyl-5-nitro-1,2,3,4-tetrahydroquinoline-1-carboxylate as a red oil (32 g, 72%). LCMS (ES, *m*/*z*): 406 [M+H]⁺.

### Step 5. methyl (2S)-5-amino-6-[[(1R,3R)-3-(methoxycarbonyl)cyclohexyl]amino]-2-methyl-1,2,3,4-tetrahydroquinoline-1-carboxylate

A solution of (2S)-6-[[(1R,3R)-3-(methoxycarbonyl)cyclohexyl]amino]-2-methyl-5-nitro-1,2,3,4-tetrahydroquinoline-1-carboxylate (31.0 g, 76.46 mmol), NH₄Cl (24.3 g, 454.28 mmol), Fe (64.3 g, 1.15 mol) in tetrahydrofuran (300 mL), ethanol (300 mL), water (100 mL) was stirred for 1 h at 80 °C and then cooled to room temperature. The solids were filtered out by filtration. The resulting solution was diluted with water (300 mL) and extracted with ethyl acetate (3 x 400 mL). The combined organic layers were dried over anhydrous sodium sulfate, filtered, and concentrated under vacuum to afford methyl (2S)-5-amino-6-[[(1R,3R)-3-(methoxycarbonyl)cyclohexyl]amino]-2-methyl-1,2,3,4-tetrahydroquinoline-1-carboxylate as a dark green solid (27.5 g, 92%). LCMS (ES, *m*/*z*): 376 [M+H]⁺.

### Step 6. methyl (2S)-5-((R)-2-hydroxy-2-phenylacetamido)-6-[[(1R,3R)-3-(methoxycarbonyl)cyclohexyl]amino]-2-methyl-1,2,3,4-tetrahydroquinoline-1-carboxylate

A solution of (R)-2-hydroxy-2-phenylacetic acid (972 mg, 6.39 mmol), HATU (1.20 g, 3.16 mmol), methyl (2S)-5-amino-6-[[(1R,3R)-3-(methoxycarbonyl)cyclohexyl]amino]-2-methyl-1,2,3,4-tetrahydroquinoline-1-carboxylate (800 mg, 2.13 mmol), DIEA (1.08 mL, 6.20 mmol) in N,N-dimethylformamide (10 mL) was stirred for 5 h at room temperature (25 °C). The resulting solution was diluted with water (30 mL), and extracted with ethyl acetate (3 x 50 mL). The organic layers were combined and washed with brine (2 x 25 mL). The combined organic layers were dried over anhydrous sodium sulfate, filtered, and concentrated under vacuum. The resulting crude product was purified by silica gel chromatography (eluting with 1:1 ethyl acetate/petroleum ether) to afford methyl (2S)-5-((R)-2-hydroxy-2-phenylacetamido)-6-[[(1R,3R)-3-(methoxycarbonyl)cyclohexyl]amino]-2-methyl-1,2,3,4-tetrahydroquinoline-1-carboxylate as a colorless oil (600 mg, 55%). LCMS (ES, *m*/*z*): 510 [M+H]⁺

### Step 7. methyl (7S)-2-[(R)-hydroxy(phenyl)methyl]-3-[(1R,3R)-3-(methoxycarbonyl)cyclohexyl]-7-methyl-3H,6H,7H,8H,9H-imidazo[4,5-f]quinoline-6-carboxylate

A solution of methyl (2S)-5-((R)-2-hydroxy-2-phenylacetamido)-6-[[(1R,3R)-3-(methoxycarbonyl)cyclohexyl]amino]-2-methyl-1,2,3,4-tetrahydroquinoline-1-carboxylate (600 mg, 1.18 mmol) in glacial acetic acid (5 mL, 98%) was stirred for overnight at 40 °C and then cooled to room temperature. The reaction mixture was diluted with water (10 mL). The pH value of the solution was adjusted to 8 with sodium bicarbonate (saturated aqueous solution). The resulting solution was extracted with ethyl acetate (3 x 15 mL). The organic layers were combined and dried over anhydrous sodium sulfate, filtered, and concentrated under vacuum. The resulting crude product was purified by silica gel chromatography (eluting with 1:1 ethyl acetate/petroleum ether) to afford methyl (7S)-2-[(R)-hydroxy(phenyl)methyl]-3-[(1R,3R)-3-(methoxycarbonyl)cyclohexyl]-7-methyl-3H,6H,7H,8H,9H-imidazo[4,5-f]quinoline-6-carboxylate(400 mg, 69%) as a colorless oil. LCMS (ES, *m*/*z*): 492 [M+H]⁺.

### Step 8. (1R,3R)-3-[(7S)-2-[(R)-hydroxy(phenyl)methyl]-6-(methoxycarbonyl)-7-methyl-3H,6H,7H,8H,9H-imidazo[4,5-f]quinolin-3-yl]cyclohexane-1-carboxylic acid

A solution of methyl (7S)-2-[(R)-hydroxy(phenyl)methyl]-3-[(1R,3R)-3-(methoxycarbonyl)cyclohexyl]-7-methyl-3H,6H,7H,8H,9H-imidazo[4,5-f]quinoline-6-carboxylate (400 mg, 0.81 mmol), LiOH (100 mg, 4.17 mmol) in tetrahydrofuran (5 mL) and water (2 mL) was stirred for overnight at room temperature (25 °C). The resulting mixture was concentrated under vacuum. The crude product was purified by Prep-HPLC (Column: XBridge Shield RP18 OBD Column, 5um, 19 x 150 mm; Mobile Phase, A: water (containing 10 mmol/L NH₄HCO₃) and B: ACN (3% to 30% over 21 min); Detector: UV 254 nm). The product fractions were lyophilized to afford (1R,3R)-3-[(7S)-2-[(R)-hydroxy(phenyl)methyl]-6-(methoxycarbonyl)-7-methyl-3H,6H,7H,8H,9H-imidazo[4,5-f]quinolin-3-yl]cyclohexane-1-carboxylic acid as a white solid (83.7 mg, 22%). Enantiomeric excess was determined via HPLC: Column: CHIRALPAK IE-3, Column size: 0.46 x 5 cm; 3 µm; Mobile phase: Hex (0.1%FA): EtOH = 85:15, Flow :1.0ml/min. ¹H-NMR (CD₃OD, 400 MHz) δ (ppm): 7.47-7.28 (m, 7H), 6.12(s, 1H), 4.84-4.74(m, 2H), 3.79(s, 3H), 3.33-3.25(m, 1H), 3.03-2.96 (m, 1H), 2.86-2.82 (m, 1H), 2.38-2.25 (m, 2H), 2.25-2.07 (m, 3H), 1.79-1.72 (m, 1H), 1.64-1.57 (m, 2H), 1.40-1.29 (m, 2H), 1.16 (d, *J* = 6.8 Hz, 3H). LCMS (ES, *m*/*z*): 478 [M+H]⁺; 99.13% ee.

### Example 4: (1R,3R)-3-[(7S)-2-[(S)-[2-(difluoromethoxy)-5-fluorophenyl](hydroxy)methyl]-6-(methoxycarbonyl)-7-methyl-3H,6H,7H,8H,9H-imidazo[4,5-f]quinolin-3-yl]cyclohexane-1-carboxylic acid (452), (1R,3R)-3-[(7S)-2-[(R)-[2-(difluoromethoxy)-5-fluorophenyl](hydroxy)methyl]-6-(methoxycarbonyl)-7-methyl-3H,6H,7H,8H,9H-imidazo[4,5-f]quinolin-3-yl]cyclohexane-1-carboxylic acid (515)

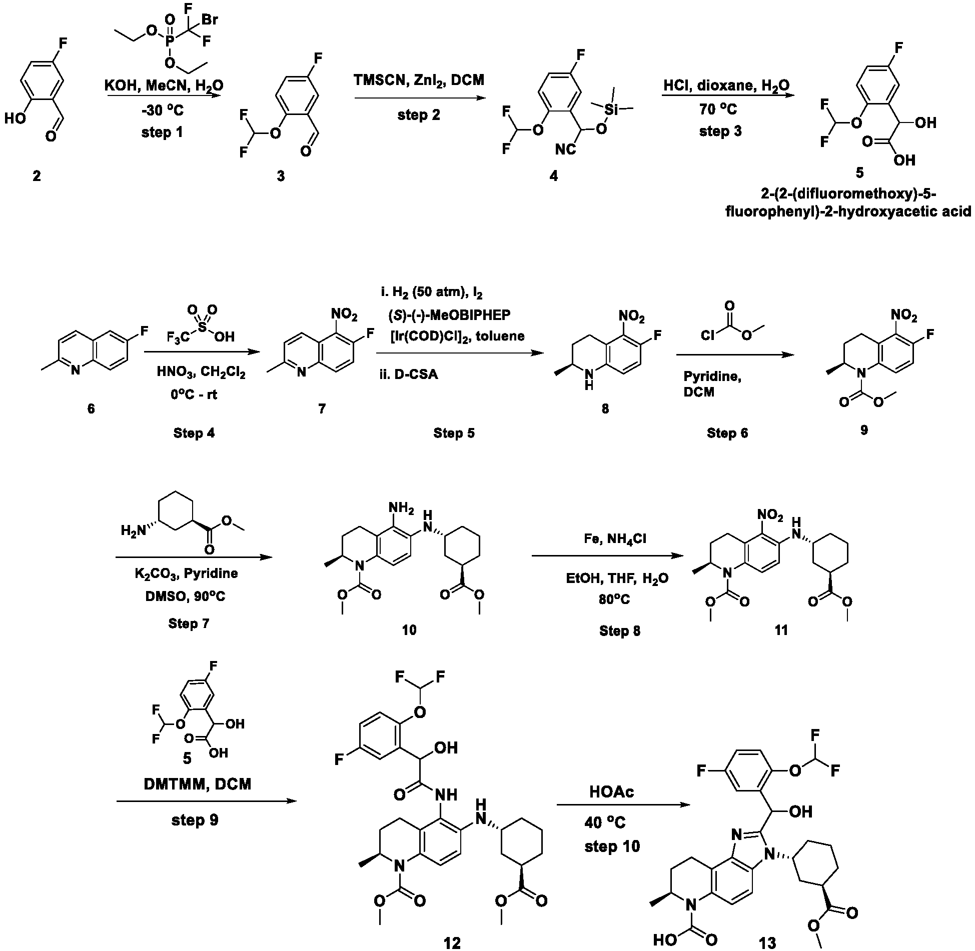

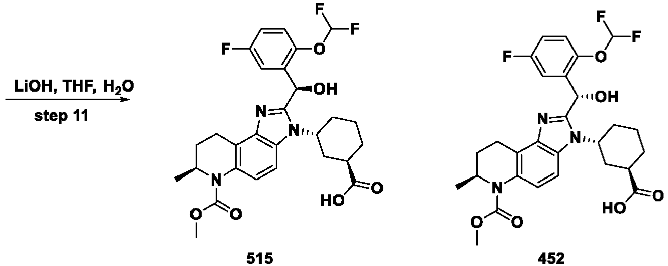

### Step 1. 2-(difluoromethoxy)-5-fluorobenzaldehyde

A solution of 5-fluoro-2-hydroxybenzaldehyde (2.0 g, 14.3 mmol), diethyl (bromodifluoromethyl)phosphonate (5.69 g, 21.3 mmol), potassium hydroxide (16.0 g, 285 mmol) in MeCN (100 mL) and water(50 mL) was stirred for 1 h at -30 °C. The reaction mixture was diluted with water (20 mL). The resulting solution was extracted with ethyl acetate (3x100 mL) and the organic layers combined and dried over anhydrous sodium sulfate. The solids were filtered out. The resulting mixture was concentrated under vacuum. The resulting crude product was purified by silica gel chromatography (eluting with 1:1 ethyl acetate/petroleum ether) to afford 2-(difluoromethoxy)-5-fluorobenzaldehyde as a yellow solid (1.46 g, 54%). LCMS (ES, *m*/*z*): 191 [M+H]⁺.

### Step 2. 2-[2-(difluoromethoxy)-5-fluorophenyl]-2-[(trimethylsilyl)oxy]acetonitrile

A solution of 2-(difluoromethoxy)-5-fluorobenzaldehyde (1.46 g, 7.68 mmol), TMSCN (760 mg, 7.66 mmol), ZnI₂ (50 mg, 0.16 mmol) in dichloromethane (3 mL) was stirred for 2 h at room temperature (25 °C). The resulting mixture was concentrated under vacuum. The resulting crude product was purified by silica gel chromatography (eluting with 1:1 ethyl acetate/petroleum ether) to afford 2-[2-(difluoromethoxy)-5-fluorophenyl]-2-[(trimethylsilyl) oxy]acetonitrile as a yellow solid (800 mg, 36%) . LCMS (ES, m/z):290 [M+H]⁺.

### Step 3. 2-[2-(difluoromethoxy)-5-fluorophenyl]-2-hydroxyacetic acid

A solution of 2-[2-(difluoromethoxy)-5-fluorophenyl]-2-[(trimethylsilyl)oxy] acetonitrile (800 mg, 2.77 mmol), 1,4-dioxane (2.0 mL), hydrogen chloride (1.0 mL, 12M) in water (2 mL) was stirred for 12 h at 70 °C and then cooled to room temperature. The resulting solution was concentrated under vacuum. The crude product was purified by reverse phase column chromatography (water (containing 0.05%TFA)/MeCN) to afford 2-[2-(difluoromethoxy)-5-fluorophenyl]-2-hydroxyacetic acid (400 mg, 61%). LCMS (ES, m/z): 237 [M+H]⁺.

### Step 4. 6-fluoro-2-methyl-5-nitroquinoline

A solution of trifluoromethanesulfonic acid (82.0 mL, 0.923 mol) in HNO₃ (19.6 mL, 0.437 mol) was stirred for 20 min at 0 °C. This was followed by the addition of 6-fluoro-2-methylquinoline (50.0 g, 0.310 mol) in dichloromethane (300 mL) at 0 °C. The resulting mixture was stirred for 15 h at room temperature (25 °C). The reaction mixture was diluted with water (300 mL). The pH value of the solution was adjusted to 8 with sodium bicarbonate (saturated aqueous solution). The resulting solution was extracted with dichloromethane (3 x 300 mL). The combined organic layers were dried over anhydrous sodium sulfate, filtered, and concentrated under vacuum. The residue was purified by silica gel chromatography (eluting with 1:4 ethyl acetate/petroleum ether) to afford 6-fluoro-2-methyl-5-nitroquinoline as a light yellow solid (60.0 g, 94%). LCMS (ES, *m*/*z*): 207 [M+H]⁺.

### Step 5. (2S)-6-fluoro-2-methyl-5-nitro-1,2,3,4-tetrahydroquinoline

A solution of (S)-(-)-MeO-BIPHEP (1.03 g, 1.77 mmol), chloro(1,5-cyclooctadiene)iridium(I) dimer (538 mg, 0.80 mmol) in toluene (100 mL) was stirred for 30 min at room temperature (25 °C) under an atmosphere of nitrogen. This was followed by the addition of I₂ (410 mg, 1.62 mmol), 6-fluoro-2-methyl-5-nitroquinoline (33.0 g, 0.160 mol) in toluene (100 mL). The resulting mixture was stirred for 20 h at room temperature (25 °C) under hydrogen (50 atm). The resulting mixture was concentrated under vacuum and purified by silica gel chromatography (eluting with 1:1 ethyl acetate/petroleum ether) to afford the crude product (35.0 g). The crude product was dissolved in ethyl acetate (230 mL), followed by the addition of D-Camphorsulfonic acid (36.9 g, 0.158 mol). The resulting solution was stirred for 1 h at 60 °C and then cooled to room temperature. The solids were collected by filtration, and rinsed with ethyl acetate (120 mL). The solids were dissolved in water (50 mL). The pH value of the solution was adjusted to 8 with sodium bicarbonate (saturated aqueous solution). The resulting solution was extracted with ethyl acetate (3 x 120 mL). The combined organic layers were dried over anhydrous sodium sulfate, filtered, and concentrated under vacuum to afford (2S)-6-fluoro-2-methyl-5-nitro-1,2,3,4-tetrahydroquinoline as a red solid (25.5 g, 76%). LCMS (ES, *m*/*z*): 211 [M+H]⁺.

### Step 6. methyl (2S)-6-fluoro-2-methyl-5-nitro-1,2,3,4-tetrahydroquinoline-1-carboxylate

A solution of (2S)-6-fluoro-2-methyl-5-nitro-1,2,3,4-tetrahydroquinoline (25.3 g, 0.120 mol), pyridine (39.0 mL, 0.484 mol), methyl carbonochloridate (18.7 mL, 0.242 mol) in dichloromethane (150 mL) was stirred for 3 h at room temperature (25 °C). The reaction was washed with 1M hydrogen chloride (2 x 70 mL). The combined organic layers were dried over anhydrous sodium sulfate, filtered, and concentrated under vacuum to afford methyl (2S)-6-fluoro-2-methyl-5-nitro-1,2,3,4-tetrahydroquinoline-1-carboxylate as a yellow solid (29.8 g, 92%). LCMS (ES, *m*/*z*): 269 [M+H]⁺.

### Step 7. methyl (2S)-6-[[(1R,3R)-3-(methoxycarbonyl)cyclohexyl]amino]-2-methyl-5-nitro-1,2,3,4-tetrahydroquinoline-1-carboxylate

A solution of methyl (2S)-6-fluoro-2-methyl-5-nitro-1,2,3,4-tetrahydroquinoline-1-carboxylate (29.6 g, 0.110 mol), pyridine (29.6 mL, 0.368 mol), potassium carbonate (30.5 g, 0.220 mol), methyl (1R,3R)-3-aminocyclohexane-1-carboxylate (25.6 g, 162.84 mmol) in DMSO (270 mL) was stirred for 15 h at 90 °C and then cooled to room temperature. The reaction was quenched by the addition of water (200 mL) and extracted with ethyl acetate (3 x 300 mL). The combined organic layers were dried over anhydrous sodium sulfate, filtered, and concentrated under vacuum. The resulting crude product was purified by silica gel chromatography (eluting with 1:1 ethyl acetate/petroleum ether) to afford methyl (2S)-6-[[(lR,3R)-3-(methoxycarbonyl)cyclohexyl] amino]-2-methyl-5-nitro-1,2,3,4-tetrahydroquinoline-1-carboxylate as a red oil (32 g, 72%). LCMS (ES, *m*/*z*): 406 [M+H]⁺.

### Step 8. methyl (2S)-5-amino-6-[[(1R,3R)-3-(methoxycarbonyl)cyclohexyl]amino]-2-methyl-1,2,3,4-tetrahydroquinoline-1-carboxylate

A solution of methyl (2S)-6-[[(lR,3R)-3-(methoxycarbonyl)cyclohexyl]amino]-2-methyl-5-nitro-1,2,3,4-tetrahydroquinoline-1-carboxylate (31.0 g, 76.46 mmol), NH₄Cl (24.3 g, 454.28 mmol), Fe (64.3 g, 1.15 mol) in tetrahydrofuran (300 mL), ethanol (300 mL), water (100 mL) was stirred for 1 h at 80 °C and then cooled to room temperature. The solids were filtered out by filtration. The resulting solution was diluted with water (300 mL) and extracted with ethyl acetate (3 x 400 mL). The combined organic layers were dried over anhydrous sodium sulfate, filtered, and concentrated under vacuum to afford methyl (2S)-5-amino-6-[[(1R,3R)-3-(methoxycarbonyl)cyclohexyl]amino]-2-methyl-1,2,3,4-tetrahydroquinoline-1-carboxylate as a dark green solid (27.5 g, 92%). LCMS (ES, *m*/*z*): 376 [M+H]⁺.

### Step 9. methyl (2S)-5-[2-[2-(difluoromethoxy)-5-fluorophenyl]-2-hydroxyacetamido]-6-[[(1R,3R)-3-(methoxycarbonyl)cyclohexyl]amino]-2-methyl-1,2,3,4-tetrahydroquinoline-1-carboxylate

A solution of methyl (2S)-5-amino-6-[[(lR,3R)-3-(methoxycarbonyl)cyclohexyl] amino]-2-methyl-1,2,3,4-tetrahydroquinoline-1-carboxylate (200 mg, 0.53 mmol), 2-[2-(difluoromethoxy)-5-fluorophenyl]-2-hydroxyacetic acid (220 mg, 0.93 mmol), DMTMM (350 mg, 1.26 mmol) in dichloromethane (5 mL) was stirred for 1 h room temperature (25 °C). The resulting solution was concentrated under vacuum. The resulting crude product was purified by silica gel chromatography (eluting with 1:1 ethyl acetate/petroleum ether) to afford methyl (2S)-5-[2-[2-(difluoromethoxy)-5-fluorophenyl]-2-hydroxyacetamido]-6-[[(lR,3R)-3-(methoxycarbonyl)cyclohexyl] amino]-2-methyl-1,2,3,4-tetrahydroquinoline-1-carboxylate as a yellow solid (70.0 mg, 22%). LCMS (ES, m/z): 594 [M+H]⁺.

### Step 10. methyl (7S)-2-[[2-(difluoromethoxy)-5-fluorophenyl](hydroxy)methyl]-3-[(1R,3R)-3-(methoxycarbonyl)cyclohexyl]-7-methyl-3H,6H,7H,8H,9H-imidazo[4,5-f]quinoline-6-carboxylate

A solution of methyl (2S)-5-[2-[2-(difluoromethoxy)-5-fluorophenyl]-2-hydroxyacetamido]-6-[[(1R,3R)-3-(methoxycarbonyl)cyclohexyl]amino]-2-methyl-1,2,3,4-tetrahydroquinoline-1-carboxylate (70.0 mg, 0.12 mmol) in glacial acetic acid (2.0 mL) was stirred for overnight at 40 °C and then cooled to room temperature. The resulting solution was concentrated under vacuum. The resulting crude product was purified by silica gel chromatography (eluting with 1:2 ethyl acetate/petroleum ether) to afford methyl (7S)-2-[[2-(difluoromethoxy)-5-fluorophenyl](hydroxy)methyl]-3-[(1R,3R)-3-(methoxycarbonyl) cyclohexyl]-7-methyl-3H,6H,7H,8H,9H-imidazo[4,5-f]quinoline-6-carboxylate as a yellow solid (50.0 mg, 74%). LCMS (ES, m/z): 576 [M+H]⁺.

### Step 11. (1R,3R)-3-[(7S)-2-[(S)-[2-(difluoromethoxy)-5-fluorophenyl](hydroxy)methyl]-6-(methoxycarbonyl)-7-methyl-3H,6H,7H,8H,9H-imidazo[4,5-f]quinolin-3-yl]cyclohexane-1-carboxylic acid; (1R,3R)-3-[(7S)-2-[(R)-[2-(difluoromethoxy)-5-fluorophenyl](hydroxy)methyl]-6-(methoxycarbonyl)-7-methyl-3H,6H,7H,8H,9H-imidazo[4,5-f]quinolin-3-yl]cyclohexane-1-carboxylic acid

A solution of methyl (7S)-2-[[2-(difluoromethoxy)-5-fluorophenyl](hydroxy)methyl]-3-[(1R,3R)-3-(methoxycarbonyl)cyclohexyl]-7-methyl-3H,6H,7H,8H,9H-imidazo[4,5-f] quinoline -6-carboxylate (50.0 mg, 0.09 mmol), LiOH (10.0 mg, 0.42 mmol) in tetrahydrofuran (2.0 mL) and water (2.0 mL) was stirred for overnight at room temperature (25 °C). The resulting mixture was concentrated under vacuum. The crude product was purified by Prep-HPLC (Column, XBridge Shield RP18 OBD Column, 30x150 mm, 5 um; Mobile phase, A: water (containing 10 mmol/L NH₄HCO₃) and B: ACN (25.0% to 35.0% over 8 min); Detector, UV 254/220 nm). The product fractions were concentrated to afford (1R,3R)-3-[(7S)-2-[(S)-[2-(difluoromethoxy)-5-fluorophenyl](hydroxy)methyl]-6-(methoxycarbonyl)-7-methyl-3H,6H,7H,8H,9H-imidazo[4,5-f]quinolin-3-yl]cyclohexane-1-carboxylic acid (452) as a white solid (4.50 mg, 9%), and (1R,3R)-3-[(7S)-2-[(R)-[2-(difluoromethoxy)-5-fluorophenyl](hydroxy)methyl]-6-(methoxycarbonyl)-7-methyl-3H,6H,7H,8H,9H-imidazo[4,5-f]quinolin-3-yl]cyclohexane-1-carboxylic acid (515) as a white solid (4.30 mg, 9%). Enantiomeric excess was determined via HPLC: Column: CHIRALPAK IE-3, Column size: 0.46 x 5 cm; 3 µm; Co-Solvent: IPA (20 mM NH₃) Gradient (B%) : 10% to 50% in 4.0min, hold 2.0 min at 50%.
**First eluting isomer (452):** ¹H-NMR (CD₃OD, 400 MHz) δ (ppm): 7.63-7.61 (m, 1H), 7.53 (d, *J* = 8.8 Hz, 1H), 7.41(d, *J* = 9.2Hz, 1H) 7.20-7.13 (m, 2H), 6.67-6.30 (m, 2H), 4.98-4.95 (m, 1H), 4.76-4.71 (m, 1H), 3.78 (s, 3H), 3.15-2.86 (m, 3H), 2.46-2.20 (m, 5H), 1.81-1.53 (m, 5H), 1.13 (d, *J* = 6.8 Hz, 3H). LCMS (ES, *m*/*z*): 562 [M+H]⁺.
**Second eluting isomer (515):** ¹H-NMR (CD₃OD, 400 MHz) δ (ppm): 7.55-7.53 (m, 1H), 7.47-7.42 (m, 2H), 7.40-7.12 (m, 2H), 6.85-6.44 (m, 2H), 4.94-4.91 (m, 1H), 4.76-4.71 (m, 1H), 3.78 (s, 3H), 3.22-2.84 (m, 3H), 2.46-2.23 (m, 5H), 1.84-1.61 (m, 5H), 1.14 (d, *J* = 6.4 Hz, 3H). LCMS (ES, *m*/*z*): 562 [M+H]⁺ ; >99.99% ee.

In some embodiments, the disclosure provides the first eluting isomer obtained from Step 11 of the process described in Example 4. In some embodiments, the disclosure provides the second eluting isomer obtained from Step 11 of the process described in Example 4.

### Example 5: HTRF biochemical assay for CBP and BRD4 activity

The ability of compounds of formula I to selectively inhibit CBP was determined using the following HTRF biochemical assay for CBP and BRD4 activity. The assay was performed in a final volume of 6 µL in assay buffer containing 50 mM Hepes (pH 7.5, (0.5M Hepes, pH 7.5 solution; Teknova H1575)), 0.5 mM GSH, 0.01% BGG (0.22 µM filtered, Sigma, G7516-25G), 0.005% BSA (0.22 µM filtered, EMD Millipore Cosporation, 126575) and 0.01% Triton X-100 (Sigma, T9284-10L). Nanoliter quantities of 10-point, 3-fold serial dilution in DMSO were pre-dispensed into 1536 assay plates (Corning, #3724BC) for a final test concentration of 33 µM to 1.7 nM, top to lowest dose, respectively. 3 µL of 2x Protein and 3 µL of 2 x Peptide Ligand were added to assay plates (pre-stamped with compound). Plates were incubated for varying times at room temperature prior to measuring the signal. TR-FRET (Time-Resolved Fluorescence Resonance Energy Transfer) was measured on a PHERAstar plate reader (BMG, equipped with HTRF optic module [337/520/490]) or on an Envision plate reader (PerkinElmer, equipped with the TRF Laser unit, TRF dual mirror D400/D505 and emission filters M520 and M495). Data were reported as percent inhibition compared with control wells based on the following equation: %inh = 1-((TR-FRET ratio - AveLow) / (AveHigh - AveLow)) where TR-FRET ratio = (Fluorescence at 520nm/Fluorescence at 490nm) * 10000), AveLow = average TR-FRET ratio of no enzyme control (n=32), and AveHigh= average TR-FRET ratio of DMSO control (n = 32). IC50 values were determined by curve fitting of the standard 4 parameter logistic fitting algorithm included in the Activity Base software package: IDBS XE Designer Model205. Data is fitted using the Levenburg Marquardt algorithm. For all assay formats data were reported as percent inhibition compared with control wells based on the following equation: %inh = 100*((FLU - AveLow) / (AveHigh - AveLow)) where FLU = measured Fluorescence, AveLow = average Fluorescence of no enzyme control (n=32), and AveHigh= average Fluorescence of DMSO control (n=32). IC50 values were determined by curve fitting of the standard 4 parameter logistic fitting algorithm included in the Activity Base software package: IDBS XE Designer Model205. Data is fitted using the Levenburg Marquardt algorithm. IC₅₀ values are shown in Figure 1. As set forth in Figure 1, an IC₅₀ value of less than or equal to 0.01 µM is marked "++++"; a value greater than 0.01 µM and less than or equal to 0.1 µM is marked "+++"; a value greater than 0.1 µM and less than or equal to 1 µM is marked "++"; and values greater than 1 µM is marked "+." Compounds that were not tested in a particular assay are marked "NT."

In some embodiments, the CBP Inhibitor Comound is also selective for CBP activity compared to BRD4 activity, as determined by obtaining a IC50 value for CBP inhibition in the HTRF biochemical assay for CBP that is lower than the corresponding IC50 value obtained for the HTRF biochemical assay for BRD4 activity according to Example 5. A CBP Inhibitor Composition can contain an amount of a compound of the disclosure, or a pharmaceutically acceptable salt thereof, and amounts of one or more stereoisomers of the compound up to amounts that retain sufficient activity of the composition with respect to CBP inhibition and selectivity for CBP over BRD4. Using the methods provided herein, CBP Inhibitor Compositions can contain 95% by HPLC or more of a compound of the disclosure, or a pharmaceutically acceptable salt thereof, and up to 5% by HPLC of one or more stereoisomers of the compound.

In a preferred embodiment, the present disclosure relates to compound 424 having an IC₅₀ value of less than or equal to 0.01 µM for the inhibition of CBP, and an IC₅₀ value of greater than 0.1 µM and less than or equal to 1 µM for the inhibition of BRD4 as determined by the HTRF biochemical assay for CBP and BRD4 activity described herein in Example 5.

In some embodiments, the present disclosure relates to a compound of Formula (II) selected from the group consisting of compound 424 and its related stereoisomers of structures (II-a) to (II-o) depicted above, having an IC₅₀ value of less than or equal to 0.01 µM for the inhibition of CBP, and an IC₅₀ value of greater than 0.1 µM and less than or equal to 1 µM for the inhibition of BRD4 as determined by the HTRF biochemical assay for CBP and BRD4 activity described herein in Example 5.

## Claims

1. A compound of Formula (I): 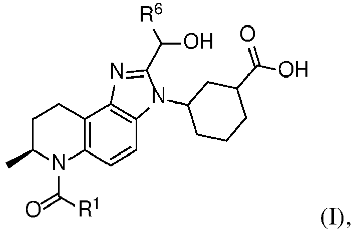 or a pharmaceutically acceptable salt, enantiomer, hydrate, solvate, stereoisomer, or tautomer thereof,
wherein:
R¹ is -OR⁵;
R⁵ is -C₁-C₆alkyl;
R⁶ is phenyl optionally substituted with one or more R¹⁰;
R¹⁰ is each independently, at each occurrence,-C₁-C₆alkyl, -C2-C6alkenyl, -C₂-C₆alkynyl, -C₃-C₈cycloalkyl, -C₄-C₈cycloalkenyl, heterocyclyl, heteroaryl, aryl, -OH, halogen,-NO₂, -CN, -NH₂, -OC₁-C₆alkyl, -OC₃-C₆cycloalkyl, -Oaryl, -Oheteroaryl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl), wherein each alkyl, alkenyl, alkynyl, cycloalkyl, cycloalkenyl, heterocyclyl, heteroaryl, or aryl is optionally substituted with one or more -R¹²;
wherein any two R¹⁰ when on non-adjacent atoms, can combine to form a bridging cycloalkyl or heterocyclyl;
wherein any two R¹⁰ when on adjacent atoms, can combine to form a cycloalkyl, heterocyclyl, aryl or heteroaryl;
R¹² is independently, at each occurrence,-C₁-C₆alkyl, -C2-C6alkenyl, -C2-C6alkynyl, -C₃-C₈cycloalkyl, -C₄-C₈cycloalkenyl, heterocyclyl, heteroaryl, aryl, -OH, halogen, oxo, -NO₂, -CN, -NH₂, -OC₁-C₆alkyl, -NHC₁-C₆alkyl, -N(C₁-C₆alkyl)₂, -S(O)₂NH(C₁-C₆alkyl), -S(O)₂N(C₁-C₆alkyl)₂, -S(O)₂C₁-C₆alkyl, -C(O)C₁-C₆alkyl, -C(O)NH₂, -C(O)NH(C₁-C₆alkyl), -C(O)N(C₁-C₆alkyl)₂, -C(O)OC₁-C₆alkyl, -N(C₁-C₆alkyl)SO₂C₁-C₆alkyl, -S(O)(C₁-C₆alkyl), -S(O)N(C₁-C₆alkyl)₂, or -N(C₁-C₆alkyl)S(O)(C₁-C₆alkyl).

2. The compound of claim 1 or a pharmaceutically acceptable salt, enantiomer, hydrate, solvate, stereoisomer, or tautomer thereof, wherein
R⁵ is -C₁-C₃alkyl;
R¹⁰ is each independently, at each occurrence halogen, -OC₁-C₆alkyl, -OC₃-C₆cycloalkyl, -Oaryl, or -Oheteroaryl, wherein each alkyl, cycloalkyl, aryl or heteroaryl is optionally substituted with one or more -R¹²;
R¹² is halogen.

3. The compound of claim 1, or a pharmaceutically acceptable salt thereof, wherein any two R¹⁰ when on non-adjacent atoms, can combine to form a bridging cycloalkyl or heterocyclyl.

4. The compound of claim 1, or a pharmaceutically acceptable salt thereof, wherein any two R¹⁰ when on adjacent atoms, can combine to form a cycloalkyl, heterocyclyl, aryl or heteroaryl.

5. The compound of claim 1, wherein R⁵ is methyl.

6. The compound of claim 5, wherein at least one R¹⁰ is -OC₁-C₆alkyl.

7. The compound of claim 5, wherein at least one R¹⁰ is -C₁-C₆alkyl.

8. The compound of claim 5, wherein at least one R¹⁰ is -OC₃-C₆cycloalkyl.

9. The compound of claim 1, or a pharmaceutically acceptable salt thereof, wherein the compound is selected from

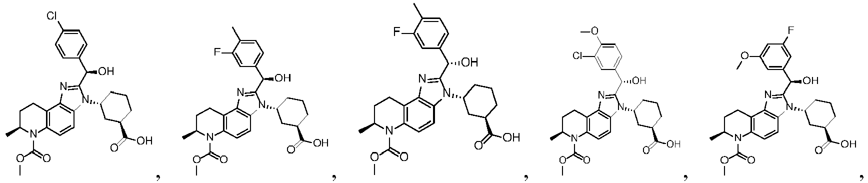

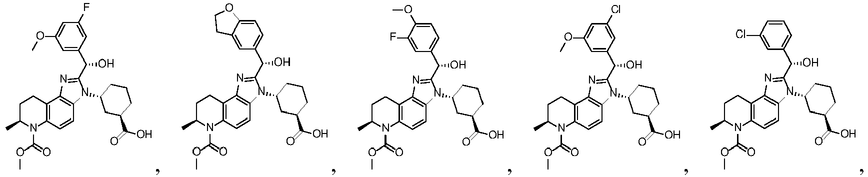

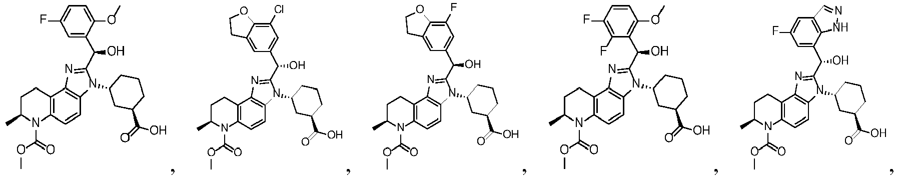

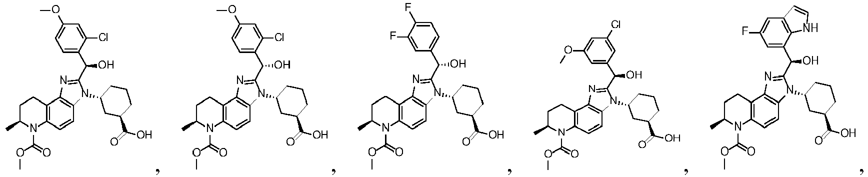

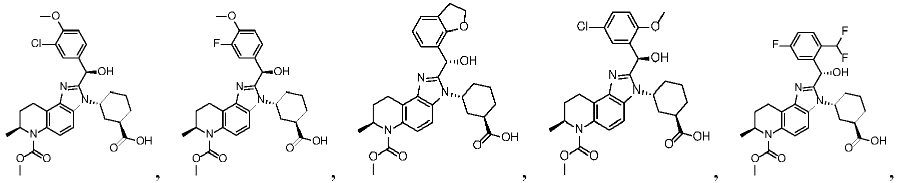

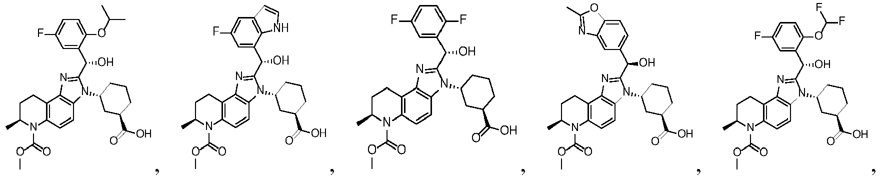

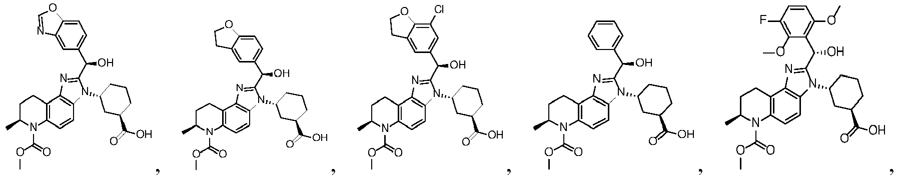

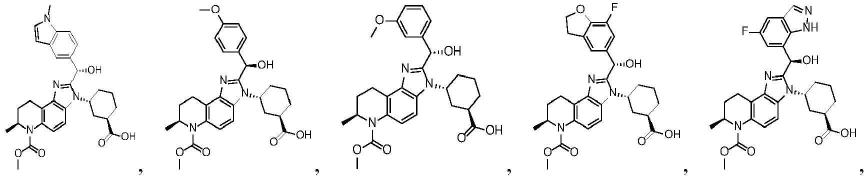

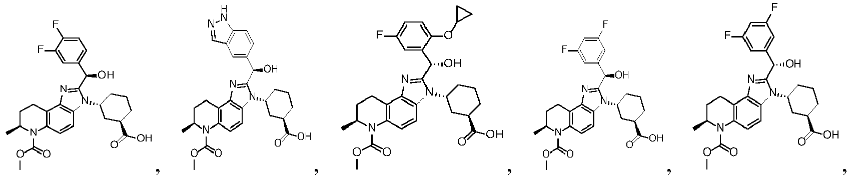

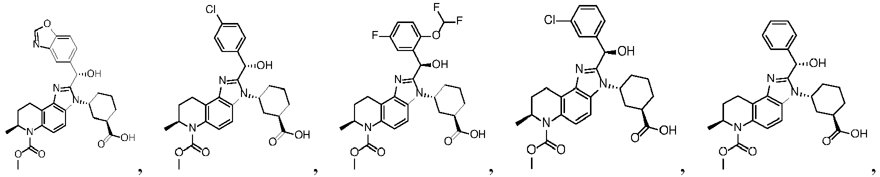

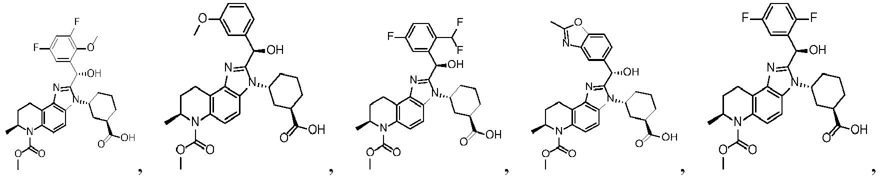

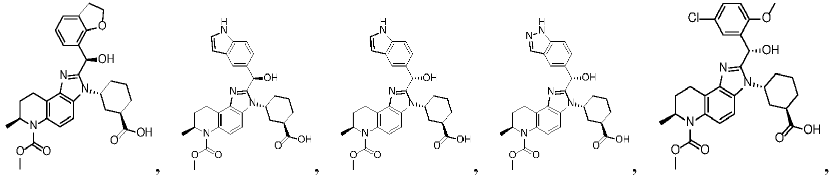

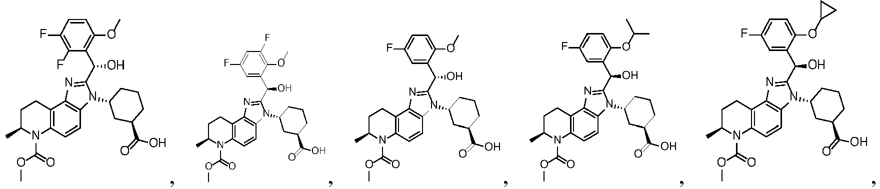

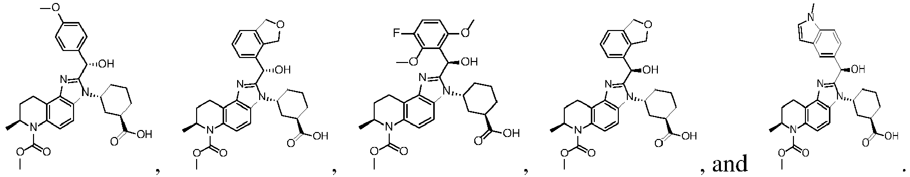

10. The compound of claim 1, or a pharmaceutically acceptable salt thereof, wherein the compound has the structure

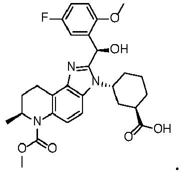

11. The compound of claim 1, or a pharmaceutically acceptable salt thereof, wherein the compound has the structure

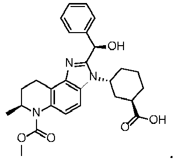

12. The compound of claim 1, or a pharmaceutically acceptable salt thereof, wherein the compound has the structure

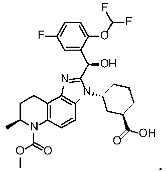

13. The compound of claim 1, or a pharmaceutically acceptable salt thereof, wherein the compound has the structure

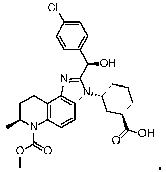

14. The compound of claim 1, or a pharmaceutically acceptable salt thereof, wherein the compound has the structure

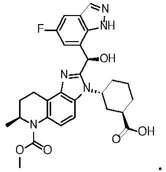

15. A pharmaceutical composition comprising a compound of any one of claims 1-14, or a pharmaceutically acceptable salt thereof, and a pharmaceutically acceptable carrier.

## Patentansprüche

1. Verbindung der Formel (I): 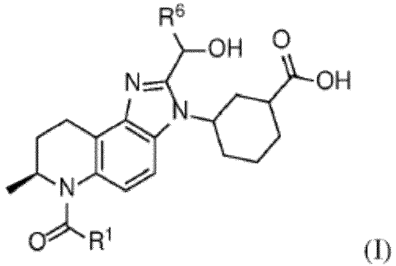 oder ein pharmazeutisch unbedenkliches Salz, Enantiomer, Hydrat, Solvat, Stereoisomer oder Tautomer davon,
wobei:
R¹ für -OR⁵ steht,
R⁵ für -C₁-C₆-Alkyl steht,
R⁶ für gegebenenfalls durch ein oder mehrere R¹⁰ substituiertes Phenyl steht,
R¹⁰ jeweils unabhängig bei jedem Auftreten für -C₁-C₆-Alkyl, -C₂-C₆-Alkenyl, -C₂-C₆-Alkinyl, -C₃-C₈-Cycloalkyl, -C₄-C₈-Cycloalkenyl, Heterocyclyl, Heteroaryl, Aryl, -OH, Halogen, -NO₂, -CN, -NH₂, -O-C₁-C₆-Alkyl, -O-C₃-C₆-Cycloalkyl, -O-Aryl, -O-Heteroaryl, -NH-C₁-C₆-Alkyl, -N (C₁-C₆-Alkyl)₂, -S(O)₂NH(C₁-C₆-Alkyl), -S(O)₂N(C₁-C₆-Alkyl)₂, -S(O)₂-C₁-C₆-Alkyl, -C(O)-C₁-C₆-Alkyl, -C(O)NH₂, -C(O)NH(C₁-C₆-Alkyl), -C(O)N(C₁-C₆-Alkyl)₂, -C(O)O-C₁-C₆-Alkyl, -N(C₁-C₆-Alkyl)-SO₂-C₁-C₆-alkyl, -S(O)(C₁-C₆-Alkyl), -S(O)N(C₁-C₆-Alkyl)₂ oder -N(C₁-C₆-Alkyl)S(O)-(C₁-C₆-alkyl) steht, wobei Alkyl, Alkenyl, Alkinyl, Cycloalkyl, Cycloalkenyl, Heterocyclyl, Heteroaryl und Aryl jeweils gegebenenfalls durch ein oder mehrere -R¹² substituiert sind,
wobei zwei beliebige R¹⁰, wenn sie sich an nicht benachbarten Atomen befinden, unter Bildung eines verbrückenden Cycloalkyl oder Heterocyclyl kombiniert sein können,
wobei zwei beliebige R¹⁰, wenn sie sich an benachbarten Atomen befinden, unter Bildung eines Cycloalkyl, Heterocyclyl, Aryl oder Heteroaryl kombiniert sein können,
R¹² unabhängig bei jedem Auftreten für -C₁-C₆-Alkyl, -C₂-C₆-Alkenyl, -C₂-C₆-Alkinyl, -C₃-C₈-Cycloalkyl, -C₄-C₈-Cycloalkenyl, Heterocyclyl, Heteroaryl, Aryl, -OH, Halogen, Oxo, -NO₂, -CN, -NH₂, -O-C₁-C₆-Alkyl, -NH-C₁-C₆-Alkyl, N(C₁-C₆-Alkyl)₂, -S(O)₂NH(C₁-C₆-Alkyl), -S(O)₂N(C₁-C₆-Alkyl)₂, -S(O)₂-C₁-C₆-Alkyl, -C(O)-C₁-C₆-Alkyl, -C(O)NH₂, -C(O)NH(C₁-C₆-Alkyl), -C(O)N(C₁-C₆-Alkyl)₂, -C(O)O-C₁-C₆-Alkyl, -N(C₁-C₆-Alkyl)-SO₂-C₁-C₆-Alkyl, -S(O)(C₁-C₆-Alkyl), -S(O)N(C₁-C₆-Alkyl)₂ oder -N(C₁-C₆-Alkyl)-S(O)-(C₁-C₆-alkyl) steht.

2. Verbindung nach Anspruch 1 oder pharmazeutisch unbedenkliches Salz, Enantiomer, Hydrat, Solvat, Stereoisomer oder Tautomer davon, wobei
R⁵ für -C₁-C₃-Alkyl steht,
R¹⁰ jeweils unabhängig bei jedem Auftreten für Halogen, -O-C₁-C₆-Alkyl, -O-C₃-C₆-Cycloalkyl, -O-Aryl oder -O-Heteroaryl steht, wobei Alkyl, Cycloalkyl, Aryl und Heteroaryl jeweils gegebenenfalls durch ein oder mehrere -R¹² substituiert sind,
R¹² für Halogen steht.

3. Verbindung nach Anspruch 1 oder pharmazeutisch unbedenkliches Salz davon, wobei zwei beliebige R¹⁰, wenn sie sich an nicht benachbarten Atomen befinden, unter Bildung eines verbrückenden Cycloalkyl oder Heterocyclyl kombiniert sein können.

4. Verbindung nach Anspruch 1 oder pharmazeutisch unbedenkliches Salz davon, wobei zwei beliebige R¹⁰, wenn sie sich an benachbarten Atomen befinden, unter Bildung eines Cycloalkyl, Heterocyclyl, Aryl oder Heteroaryl kombiniert sein können.

5. Verbindung nach Anspruch 1, wobei R⁵ für Methyl steht.

6. Verbindung nach Anspruch 5, wobei mindestens ein R¹⁰ für -O-C₁-C₆-Alkyl steht.

7. Verbindung nach Anspruch 5, wobei mindestens ein R¹⁰ für -C₁-C₆-Alkyl steht.

8. Verbindung nach Anspruch 5, wobei mindestens ein R¹⁰ für -O-C₃-C₆-Cycloalkyl steht.

9. Verbindung nach Anspruch 1 oder pharmazeutisch unbedenkliches Salz davon, wobei die Verbindung ausgewählt ist aus 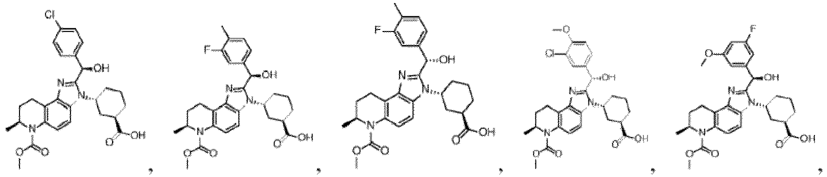 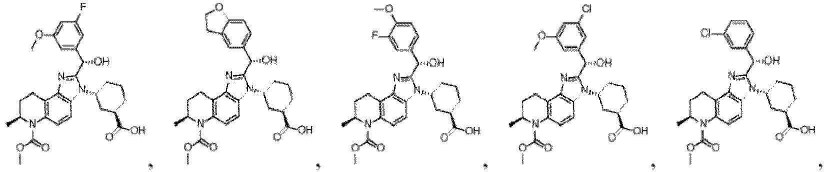 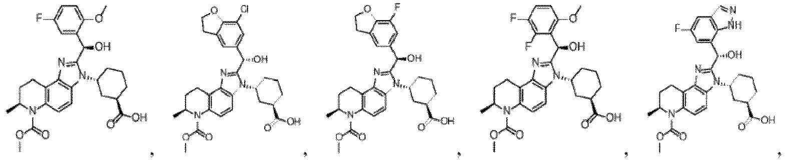 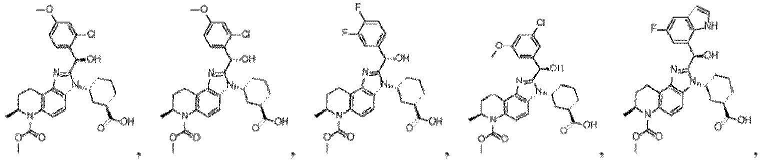 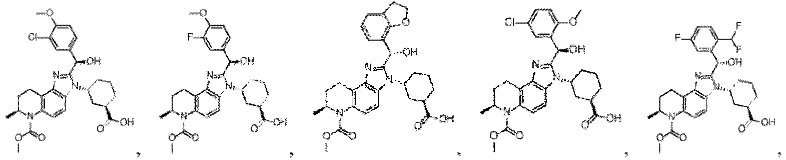 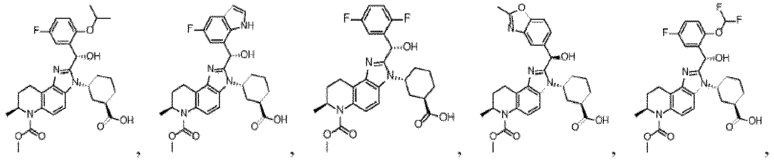 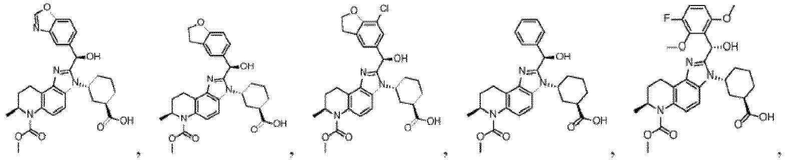 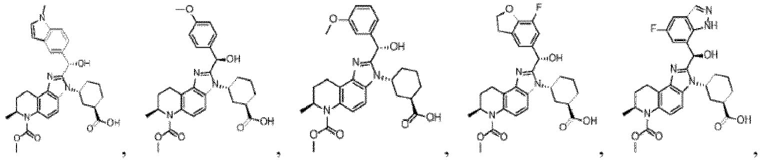 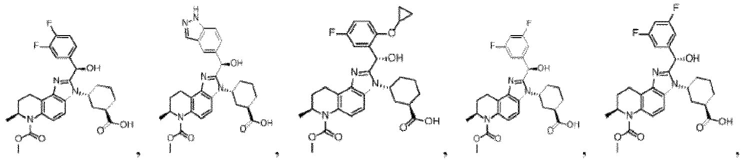 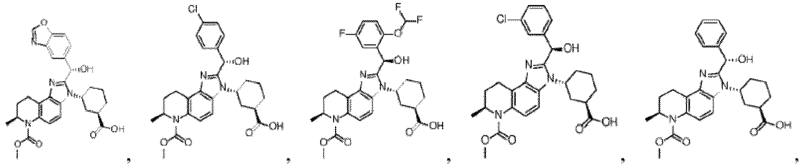 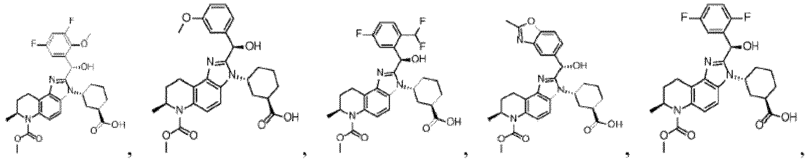 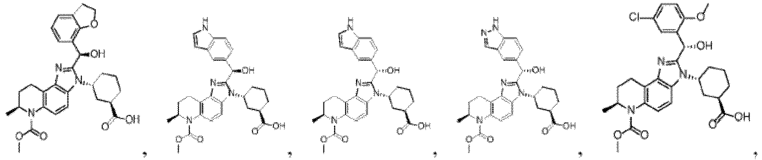 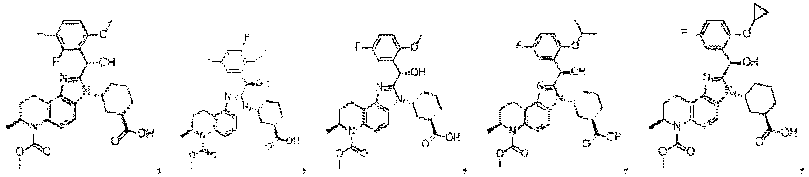 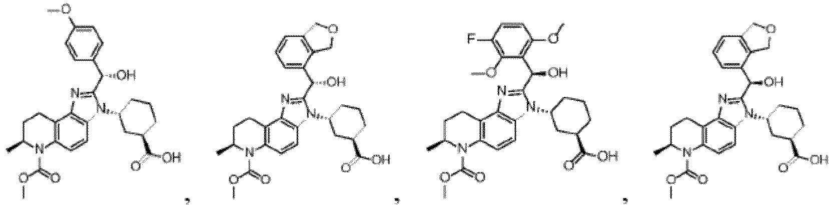 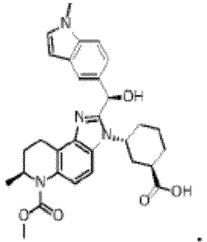 und

10. Verbindung nach Anspruch 1 oder pharmazeutisch unbedenkliches Salz davon, wobei die Verbindung die folgende Struktur aufweist:

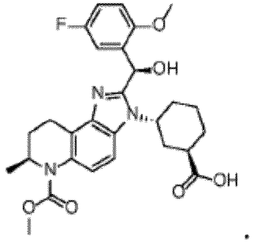

11. Verbindung nach Anspruch 1 oder pharmazeutisch unbedenkliches Salz davon, wobei die Verbindung die folgende Struktur aufweist:

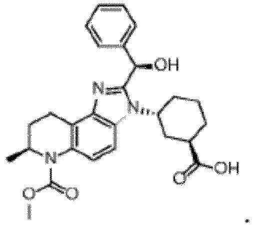

12. Verbindung nach Anspruch 1 oder pharmazeutisch unbedenkliches Salz davon, wobei die Verbindung die folgende Struktur aufweist:

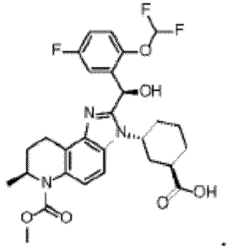

13. Verbindung nach Anspruch 1 oder pharmazeutisch unbedenkliches Salz davon, wobei die Verbindung die folgende Struktur aufweist:

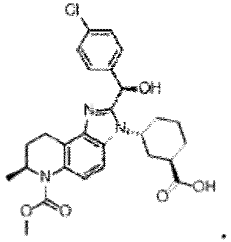

14. Verbindung nach Anspruch 1 oder pharmazeutisch unbedenkliches Salz davon, wobei die Verbindung die folgende Struktur aufweist:

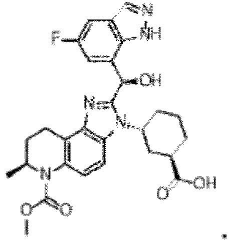

15. Pharmazeutische Zusammensetzung, umfassend eine Verbindung nach einem der Ansprüche 1 bis 14 oder ein pharmazeutisch unbedenkliches Salz davon und einen pharmazeutisch unbedenklichen Träger.

## Revendications

1. Composé de Formule (I) : 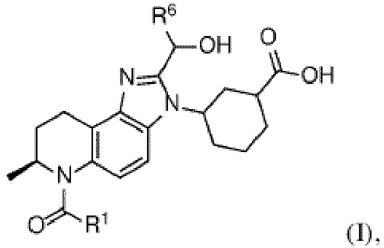 ou sel, énantiomère, hydrate, solvate, stéréoisomère, ou forme tautomère pharmaceutiquement acceptable correspondant(e),
R¹ étant -OR⁵ ;
R⁵ étant -C₁-C₆alkyle ;
R⁶ étant phényle éventuellement substitué par un ou plusieurs R¹⁰ ;
R¹⁰ étant chacun indépendamment, en chaque occurrence, -C₁-C₆alkyle, -C₂-C₆alcényle, -C₂-C₆alcynyle, -C₃-C₈cycloalkyle, -C₄-C₈cycloalcényle, hétérocyclyle, hétéroaryle, aryle, -OH, halogène, -NO₂, -CN, -NH₂, - OC₁-C₆alkyle, -OC₃-C₆cycloalkyle, -Oaryle, -Ohétéroaryle, -NHC₁-C₆alkyle, -N(C₁-C₆alkyle)₂, -S(O)₂NH(C₁-C₆alkyle), - S(O)₂N(C₁-C₆alkyle)₂, -S(O)₂C₁-C₆alkyle, -C(O)C₁-C₆alkyle, -C(O)NH₂, -C(O)NH(C₁-C₆alkyle), -C(O)N(C₁-C₆alkyle)₂, - C(O)OC₁-C₆alkyle, -N(C₁-C₆alkyl) SO₂C₁-C₆alkyle, -S(O)(C₁-C₆alkyle), -S(O)N(C₁-C₆alkyle)₂, ou -N(C₁-C₆alkyl)S(O)(C₁-C₆alkyle), chaque alkyle, alcényle, alcynyle, cycloalkyle, cycloalcényle, hétérocyclyle, hétéroaryle, ou aryle étant éventuellement substitué par un ou plusieurs -R¹² ;
deux R¹⁰ quelconques, lorsqu'ils sont sur des atomes non adjacents, pouvant se combiner pour former un cycloalkyle ou hétérocyclyle pontant ;
deux R¹⁰ quelconques, lorsqu'ils sont sur des atomes adjacents, pouvant se combiner pour former un cycloalkyle, un hétérocyclyle, un aryle ou un hétéroaryle ;
R¹² étant indépendamment, en chaque occurrence, - C₁-C₆alkyle, -C₂-C₆alcényle, -C₂-C₆alcynyle, -C₃-C₈cycloalkyle, -C₄-C₈cycloalcényle, hétérocyclyle, hétéroaryle, aryle, -OH, halogène, oxo, -NO₂, -CN, -NH₂, -OC₁-C₆alkyle, -NHC₁-C₆alkyle, -N(C₁-C₆alkyle)₂, - S(O)₂NH(C₁-C₆alkyle), -S(O)₂N(C₁-C₆alkyle)₂, -S(O)₂C₁-C₆alkyle, -C(O)C₁-C₆alkyle, -C(O)NH₂, -C(O)NH(C₁-C₆alkyle), -C(O)N(C₁-C₆alkyle)₂, -C(O)OC₁-C₆alkyle, -N(C₁-C₆alkyl)SO₂C₁-C₆alkyle, -S(O)(C₁-C₆alkyle), -S(O)N(C₁-C₆alkyle)₂, ou -N(C₁-C₆alkyl)S(O)(C₁-C₆alkyle).

2. Composé selon la revendication 1 ou sel, énantiomère, hydrate, solvate, stéréoisomère ou forme tautomère pharmaceutiquement acceptable correspondant(e),
R⁵ étant -C₁-C₃alkyle ;
R¹⁰ étant chacun indépendamment, en chaque occurrence halogène ou -OC₁-C₆alkyle, -OC₃-C₆cycloalkyle, -Oaryle, - Ohétéroaryle, chaque alkyle, cycloalkyle, aryle ou hétéroaryle étant éventuellement substitué par un ou plusieurs -R¹² ;
R¹² étant halogène.

3. Composé selon la revendication 1 ou sel pharmaceutiquement acceptable correspondant, deux R¹⁰ quelconques, lorsqu'ils sont sur des atomes non adjacents, pouvant se combiner pour former un cycloalkyle ou hétérocyclyle pontant.

4. Composé selon la revendication 1 ou sel pharmaceutiquement acceptable correspondant, deux R¹⁰ quelconques, lorsqu'ils sont sur des atomes adjacents, pouvant se combiner pour former un cycloalkyle, un hétérocyclyle, un aryle ou un hétéroaryle.

5. Composé selon la revendication 1, R⁵ étant méthyle.

6. Composé selon la revendication 5, au moins un R¹⁰ étant -OC₁-C₆alkyle.

7. Composé selon la revendication 5, au moins un R¹⁰ étant -C₁-C₆alkyle.

8. Composé selon la revendication 5, au moins un R¹⁰ étant -OC₃-C₆cycloalkyle.

9. Composé selon la revendication 1, ou sel pharmaceutiquement acceptable correspondant, le composé étant choisi parmi

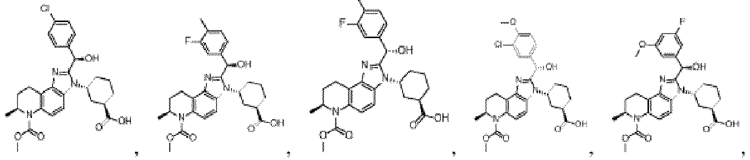

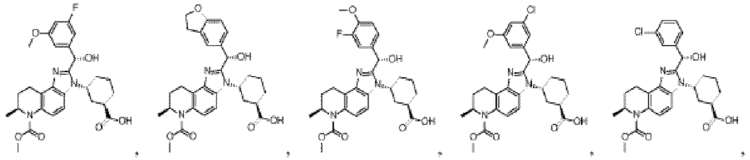

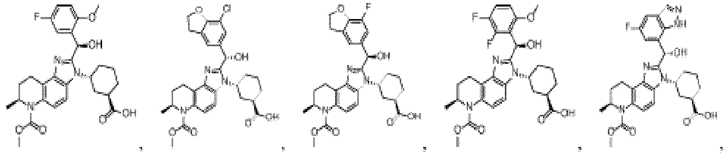

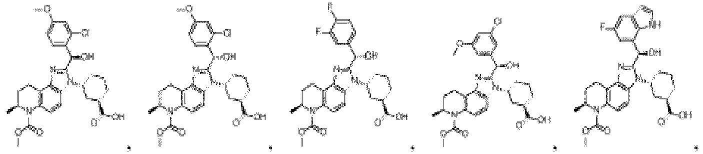

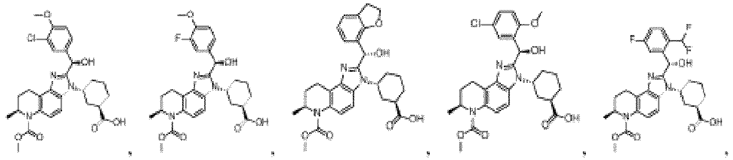

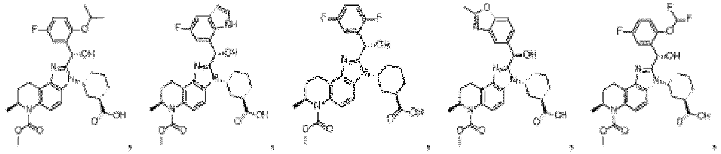

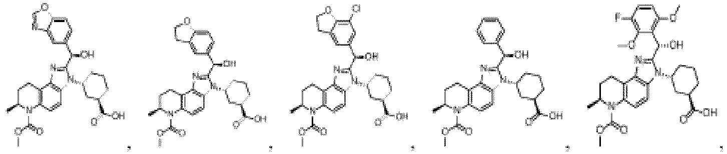

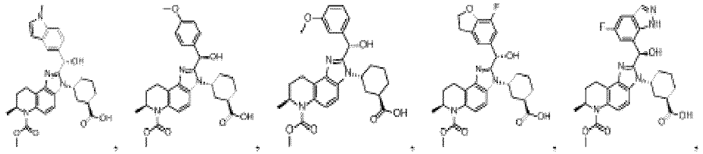

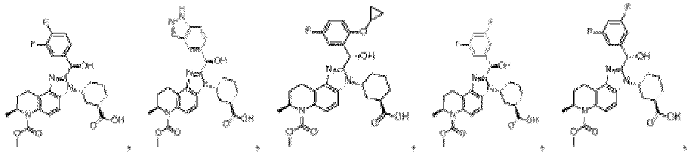

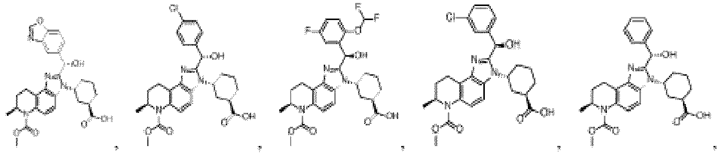

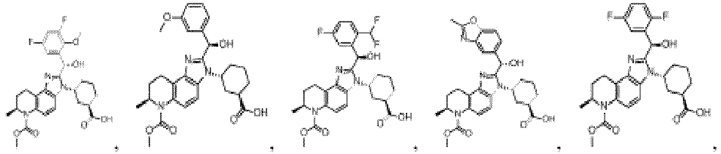

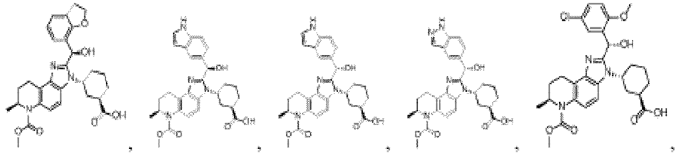

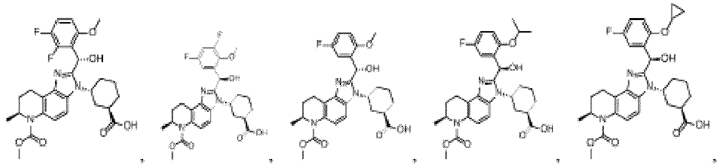

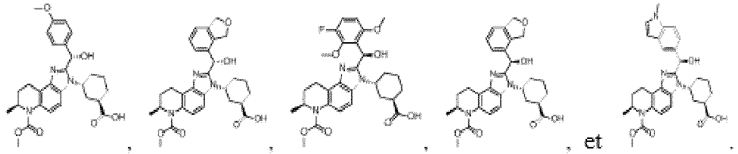

10. Composé selon la revendication 1, ou sel pharmaceutiquement acceptable correspondant, le composé possédant la structure

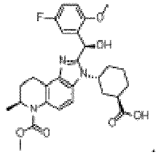

11. Composé selon la revendication 1, ou sel pharmaceutiquement acceptable correspondant, le composé possédant la structure

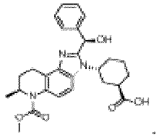

12. Composé selon la revendication 1, ou sel pharmaceutiquement acceptable correspondant, le composé possédant la structure

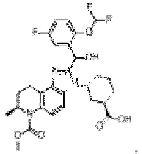

13. Composé selon la revendication 1, ou sel pharmaceutiquement acceptable correspondant, le composé possédant la structure

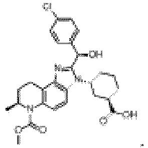

14. Composé selon la revendication 1, ou sel pharmaceutiquement acceptable correspondant, le composé possédant la structure

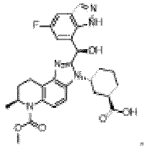

15. Composition pharmaceutique comprenant un composé selon l'une quelconque des revendications 1 à 14, ou un sel pharmaceutiquement acceptable correspondant, et un support pharmaceutiquement acceptable.
